# EUROPEAN PATENT SPECIFICATION

(11) **EP 0 726 758 B1**
(45) Date of publication and mention of the grant of the patent: **06.11.2002**
(21) Application number: 94925160.7
(22) Date of filing: 01.08.1994
(51) Int. Cl.: A61K 7/46, A61K 39/12, A01N 37/08, C07H 17/00, C07H 19/00, C07H 21/00, C12P 21/06, C12N 5/00

(54) **PEPTIDES FOR INDUCING CYTOTOXIC T LYMPHOCYTE RESPONSES TO HEPATITIS B VIRUS**
PEPTIDE ZUM INDUZIEREN EINER ANTWORT DER ZYTOTOXISCHEN T-LYMPHOZYTHEN GERICHETETGEGEN DAS HEPATITIS B-VIRUS
PEPTIDES PERMETTANT D'INDUIRE DES REPONSES DES LYMPHOCYTES T CYTOTOXIQUES AU VIRUS DE L'HEPATITE B

(30) Priority: 02.08.1993 US 100870
(43) Date of publication of application: 21.08.1996
(73) Proprietor: THE SCRIPPS RESEARCH INSTITUTE, La Jolla, CA 92037 (US)
(72) Inventor: CHISARI, Francis, V., Del Mar, CA 92014 (US)
(74) Representative: Kiddle, Simon John
(86) International application number: US9408685
(87) International publication number: WO95003777

(56) References cited:
- WO-A-93/03753
- WO-A-94/03205
- WO-A-94/20127
- J. EXP. MED., Volume 167, issued June 1988, CARBONE et al., "Induction of Cytotoxic T Lymphocytes by Primary in Vitro Stimulation with Peptides", pages 1767-1779.
- JOURNAL OF VIROLOGY, Volume 62, Number 12, issued December 1988, MACK et al., "Hepatitis B Virus Particles Contain a Polypeptide Encoded by the Largest Open Reading Frame: A Putative Reverse Transcriptase", pages 4786-4790.
- NATURE, Volume 281, issued 25 October 1979, GALIBERT et al., "Nucleotide Sequence of the Hepatitis B Virus Genome (subtype ayw), Cloned in E. Coli", pages 646-650.
- MICROBIAL PATHOGENESIS, Volume 6, issued 1989, F. CHISARI et al., "Mini Review. Hepatitis B Virus Structure and Biology", pages 311-325.

### SEQUENCE LISTING

(1) GENERAL INFORMATION:
   (i) APPLICANT:
      (A) NAME: The Scripps Research Institute
      (B) STREET: 10666 North Torrey Pines Road
      (C) CITY: La Jolla
      (D) STATE: California
      (E) COUNTRY: United States of America
      (F) POSTAL CODE (ZIP): 92037
      (G) TELEPHONE: (206) 467-9600
      (H) TELEFAX: (415) 543-5043
      (I) TELEX:
   (ii) TITLE OF INVENTION: PEPTIDES FOR INDUCING CYTOTOXIC T LYMPHOCYTE RESPONSES TO HEPATITIS B VIRUS
   (iii) NUMBER OF SEQUENCES: 44
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (v) CURRENT APPLICATION DATA:
      (A) APPLICATION NUMBER: WO
      (B) FILING DATE: 01-AUG-1994
      (C) CLASSIFICATION:
   (vi) PRIOR APPLICATION DATA:
      (A) APPLICATION NUMBER: US 08/100.870
      (B) FILING DATE: 02-AUG-1993
   (vi) PRIOR APPLICATION DATA:
      (A) APPLICATION NUMBER: US 07/935,898
      (B) FILING DATE: 26-AUG-1992
   (vi) PRIOR APPLICATION DATA:
      (A) APPLICATION NUMBER: US 07/749,540
      (B) FILING DATE: 26-AUG-1991
   (vii) ATTORNEY/AGENT INFORMATION:
      (A) NAME: Parmelee, Steven W.
      (B) REGISTRATION NUMBER: 31,990
      (C) REFERENCE/DOCKET NUMBER: 14740-2-2
   (viii) TELECOMMUNICATION INFORMATION:
      (A) TELEPHONE: (206) 467-9600
      (B) TELEFAX: (415) 543-5043
(2) INFORMATION FOR SEQ ID NO:1:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
   Gly Leu Tyr Ser Ser Thr Val Pro Val
    1               5
(2) INFORMATION FOR SEQ ID NO:2:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Gly Leu Ser Arg Tyr Val Ala Arg Leu
   1               5
(2) INFORMATION FOR SEQ ID NO:3:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Ile Leu Arg Gly Thr Ser Phe Val Tyr Val
   1               5                   10
(2) INFORMATION FOR SEQ ID NO:4:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Ser Leu Tyr Ala Asp Ser Pro Ser Val
   1               5
(2) INFORMATION FOR SEQ ID NO:5:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:5:
   Phe Leu Leu Ser Leu Gly Ile His Leu
   1 5
(2) INFORMATION FOR SEQ ID NO:6:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:6:
   Met Asp Ile Asp Pro Tyr Lys Glu Phe Gly Ala Thr Val Glu Leu Leu
   1 5 10 15
   Ser Phe Leu Pro
   20
(2) INFORMATION FOR SEQ ID NO:7:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:7:
   Pro His His Tyr Ala Leu Arg Gln Ala Ile Leu Cys Trp Gly Glu Leu
   1 5 10 15
   Met Tyr Leu Ala
   20
(2) INFORMATION FOR SEQ ID NO:8:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:8:
   Leu Leu Trp Phe His Ile Ser Cys Leu Thr Phe Gly Arg Glu Thr Val
   1               5                   10                  15
   Ile Glu Tyr Leu
               20
(2) INFORMATION FOR SEQ ID NO:9:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Glu Tyr Leu Val Ser Phe Gly Val Trp Ile Arg Thr Pro Pro Ala
   1               5                   10                  15
(2) INFORMATION FOR SEQ ID NO:10:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:10:
(2) INFORMATION FOR SEQ ID NO:11:
   (i) SEQUENCE CHARACTERISTICS:
      (A) LENGTH: 845 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:11:
   Met Pro Leu Ser Tyr Gln His Phe Arg Lys Leu Leu Leu Leu Asp Asp
   1               5                   10                  15
   Gly Thr Glu Ala Gly Pro Leu Glu Glu Glu Leu Pro Arg Leu Ala Asp
               20                  25                  30
   Glu Gly Leu Asn Arg Arg Val Ala Glu Asp Leu Asn Leu Gly Asn Leu
           35                  40                  45
   Asn Val Ser Ile Pro Trp Thr His Lys Val Gly Asn Phe Thr Gly Leu
       50                  55                  60
   Tyr Ser Ser Thr Val Pro Val Phe Asn Pro Glu Trp Gln Thr Pro Ser
   65                  70                  75                  80
   Phe Pro Asn Ile His Leu Gln Glu Asp Ile Ile Asn Arg Cys Gln Gln
                   85                  90                  95
   Phe Val Gly Pro Leu Thr Val Asn Glu Lys Arg Arg Leu Lys Leu Ile
               100                 105                 110
   Met Pro Ala Arg Phe Tyr Pro Asn Val Thr Lys Tyr Leu Pro Leu Asp
           115                 120                 125
   Lys Gly Ile Lys Pro Tyr Tyr Pro Glu His Leu Val Asn His Tyr Phe
       130                 135                 140
   Gln Thr Arg His Tyr Leu His Thr Leu Trp Lys Ala Gly Ile Leu Tyr
   145 150 155 160
   Lys Arg Glu Thr Thr Arg Ser Ala Ser Phe Cys Gly Ser Pro Tyr Ser
   165 170 175
   Trp Glu Gln Glu Leu Gln His Gly Arg Leu Val Phe Gln Thr Ser Thr
   180 185 190
   Arg His Gly Asp Glu Ser Phe Cys Ser Gln Ser Ser Gly Ile Leu Ser
   195 200 205
   Arg Ser Pro Val Gly Pro Cys Ile Arg Ser Gln Leu Arg Gln Ser Arg
   210 215 220
   Leu Gly Leu Gln Pro Gln Gln Gly His Leu Ala Arg Arg Gln Gln Gly
   225 230 235 240
   Arg Ser Gly Ser Ile Arg Ala Arg Val His Pro Thr Thr Arg Arg Ser
   245 250 255
   Phe Gly Val Glu Pro Ser Gly Ser Gly His Ile Asp Asn Ser Ala Ser
   260 265 270
   Ser Ser Ser Ser Cys Leu His Gln Ser Ala Val Arg Lys Ala Ala Tyr
   275 280 285
   Ser His Leu Ser Thr Ser Lys Arg Gln Ser Ser Ser Gly His Ala Val
   290 295 300
   Glu Leu His Asn Ile Pro Pro Asn Ser Ala Arg Ser Gln Ser Glu Gly
   305 310 315 320
   Pro Val Phe Ser Cys Trp Trp Leu Gln Phe Arg Asn Ser Lys Pro Cys
   325 330 335
   Ser Asp Tyr Cys Leu Thr His Ile Val Asn Leu Leu Glu Asp Trp Gly
   340 345 350
   Pro Cys Thr Glu His Gly Glu His Asn Ile Arg Ile Pro Arg Thr Pro
   355 360 365
   Ala Arg Val Thr Gly Gly Val Phe Leu Val Asp Lys Asn Pro His Asn
   370 375 380
   Thr Thr Glu Ser Arg Leu Val Val Asp Phe Ser Gln Phe Ser Arg Gly
   385 390 395 400
   Ser Thr Arg Val Ser Trp Pro Lys Phe Ala Val Pro Asn Leu Gln Ser
   405 410 415
   Leu Thr Asn Leu Leu Ser Ser Asn Leu Ser Trp Leu Ser Leu Asp Val
   420 425 430
   Ser Ala Ala Phe Tyr His Ile Pro Leu His Pro Ala Ala Met Pro His
   435 440 445
   Leu Leu Val Gly Ser Ser Gly Leu Ser Arg Tyr Val Ala Arg Leu Ser
   450 455 460
   Ser Asn Ser Arg Ile Ile Asn Tyr Gln His Gly Thr Met Gln Asn Leu
   465 470 475 480
   His Asp Ser Cys Ser Arg Asn Leu Tyr Val Ser Leu Leu Leu Leu Tyr
   485 490 495
   Lys Thr Phe Gly Arg Lys Leu His Leu Tyr Ser His Pro Ile Ile Leu
   500 505 510
   Gly Phe Arg Lys Ile Pro Met Gly Val Gly Leu Ser Pro Phe Leu Leu
               515                 520                 525
   Ala Gln Phe Thr Ser Ala Ile Cys Ser Val Val Arg Arg Ala Phe Pro
       530                 535                 540
   His Cys Leu Ala Phe Ser Tyr Met Asp Asp Val Val Leu Gly Ala Lys
   545                 550                 555                 560
   Ser Val Gln His Leu Glu Ser Leu Phe Thr Ala Val Thr Asn Phe Leu
                   565                 570                 575
   Leu Ser Leu Gly Ile His Leu Asn Pro Asn Lys Thr Lys Arg Trp Gly
               580                 585                 590
   Tyr Ser Leu Asn Phe Met Gly Tyr Val Ile Gly Ser Trp Gly Thr Ile
           595                 600                 605
   Pro Gln Glu His Ile Val Gln Lys Ile Lys Gln Cys Phe Arg Lys Leu
       610                 615                 620
   Pro Val Asn Arg Pro Ile Asp Trp Lys Val Cys Gln Arg Ile Val Gly
   625                 630                 635                 640
   Leu Leu Gly Phe Ala Ala Pro Phe Thr Gln Cys Gly Tyr Pro Ala Leu
                   645                 650                 655
   Met Pro Leu Tyr Ala Cys Ile Gln Ser Lys Gln Ala Phe Thr Phe Ser
               660                 665                 670
   Pro Thr Tyr Lys Ala Phe Leu Cys Lys Gln Tyr Leu Asn Leu Tyr Pro
           675                 680                 685
   Val Ala Arg Gln Arg Pro Gly Leu Cys Gln Val Phe Ala Asp Ala Thr
       690                 695                 700
   Pro Thr Gly Trp Gly Leu Ala Ile Gly His Gln Arg Met Arg Gly Thr
   705                 710                 715                 720
   Phe Val Ala Pro Leu Pro Ile His Thr Ala Glu Leu Leu Ala Ala Cys
                   725                 730                 735
   Phe Ala Arg Ser Arg Ser Gly Ala Lys Leu Ile Gly Thr Asp Asn Ser
               740                 745                 750
   Val Val Leu Ser Arg Lys Tyr Thr Ser Phe Pro Trp Leu Leu Gly Cys
           755                 760                 765
   Ala Ala Asn Trp Ile Leu Arg Gly Thr Ser Phe Val Tyr Val Pro Ser
       770                 775                 780
   Ala Leu Asn Pro Ala Asp Asp Pro Ser Arg Gly Arg Leu Gly Leu Tyr
   785                 790                 795                 800
   Arg Pro Leu Leu Arg Leu Pro Phe Arg Pro Thr Thr Gly Arg Thr Ser
                   805                 810                 815
   Leu Tyr Ala Val Ser Pro Ser Val Pro Ser His Leu Pro Asp Arg Val
               820                 825                 830
   His Phe Ala Ser Pro Leu His Val Ala Trp Arg Pro Pro
           835                 840                 845
(2) INFORMATION FOR SEQ ID NO:12:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:12:
   Ser Tyr Gln His Phe Arg Lys Leu Leu Leu
   1               5                   10
(2) INFORMATION FOR SEQ ID NO:13:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:13:
   Arg Leu Lys Leu Ile Met Pro Ala Arg
   1               5
(2) INFORMATION FOR SEQ ID NO:14:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:14:
   Val Val Asn His Tyr Phe Gln Thr Arg
   1               5
(2) INFORMATION FOR SEQ ID NO:15:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:15:
   His Thr Leu Trp Lys Ala Gly Ile Leu Tyr
   1 5 10
(2) INFORMATION FOR SEQ ID NO:16:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:16:
   Thr Leu Trp Lys Ala Gly Ile Leu Tyr Lys
   1 5 10
(2) INFORMATION FOR SEQ ID NO:17:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:17:
   Leu Tyr Ser His Pro Ile Ile Leu Gly Phe
   1 5 10
(2) INFORMATION FOR SEQ ID NO:18:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:18:
   Tyr Met Asp Asp Val Val Leu Gly Ala
   1 5
(2) INFORMATION FOR SEQ ID NO:19:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:19:
   Phe Leu Leu Ser Leu Gly Ile His Leu
   1               5
(2) INFORMATION FOR SEQ ID NO:20:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:20:
   Ala Leu Met Pro Leu Tyr Ala Cys Ile
   1               5
(2) INFORMATION FOR SEQ ID NO:21:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:21:
   Gly Thr Asp Asn Ser Val Val Leu Ser Arg
   1               5                   10
(2) INFORMATION FOR SEQ ID NO:22:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:22:
   Lys Tyr Thr Ser Phe Pro Trp Leu Leu
   1               5
(2) INFORMATION FOR SEQ ID NO:23:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:23:
   Trp Ile Leu Arg Gly Thr Ser Phe Arg
   1               5
(2) INFORMATION FOR SEQ ID NO:24:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:24:
   Asp Leu Asn Leu Gly Asn Leu Asn Val
   1               5
(2) INFORMATION FOR SEQ ID NO:25:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:25:
   Phe Leu Pro Ser Asp Tyr Phe Pro Ser Val
   1               5                   10
(2) INFORMATION FOR SEQ ID NO:26:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:26:
   Leu Leu Ala Gln Phe Thr Ser Ala Ile
   1               5
(2) INFORMATION FOR SEQ ID NO:27:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:27:
   Lys Leu His Leu Tyr Ser His Pro Ile
   1               5
(2) INFORMATION FOR SEQ ID NO:28
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:28:
   Ala Leu Met Pro Leu Tyr Ala Cys Ile
   1               5
(2) INFORMATION FOR SEQ ID NO:29:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:29:
   His Leu Tyr Ser His Pro Ile Ile Leu
   1               5
(2) INFORMATION FOR SEQ ID NO:30:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:30:
   Phe Leu Leu Ala Gln Phe Thr Ser Ala Ile
   1               5                   10
(2) INFORMATION FOR SEQ ID NO:31:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:31:
   Tyr Leu His Thr Leu Trp Lys Ala Gly Ile
   1               5                   10
(2) INFORMATION FOR SEQ ID NO:32:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:32:
   Trp Ile Leu Arg Gly Thr Ser Phe Val
   1               5
(2) INFORMATION FOR SEQ ID NO:33:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:33:
   Leu Leu Gly Cys Ala Ala Asn Trp Ile Leu
   1               5                   10
(2) INFORMATION FOR SEQ ID NO:34:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:34:
   Asn Leu Ser Trp Leu Ser Leu Asp Val
   1               5
(2) INFORMATION FOR SEQ ID NO:35:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:35:
   Phe Leu Pro Ser Asp Phe Pro Pro Ser Val
   1               5                   10
(2) INFORMATION FOR SEQ ID NO:36:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:36:
   Trp Leu Ser Leu Leu Val Pro Phe Val
   1               5
(2) INFORMATION FOR SEQ ID NO:37:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:37:
   Lys Leu Val Ala Leu Gly Ile Asn Ala Val
   1               5                   10
(2) INFORMATION FOR SEQ ID NO:38:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:38:
   Gly Leu Pro Arg Tyr Val Ala Arg Leu
   1               5
(2) INFORMATION FOR SEQ ID NO:39:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:39:
   Ser Gly Leu Ser Arg Tyr Val Ala Arg Leu
   1               5                   10
(2) INFORMATION FOR SEQ ID NO:40:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:40:
   Gly Leu Ser Arg Tyr Val Ala Arg Leu Ser
   1               5                   10
(2) INFORMATION FOR SEQ ID NO:41:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:41:
   Gly Val Ser Arg Tyr Val Ala Arg Leu
   1               5
(2) INFORMATION FOR SEQ ID NO:42:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:42:
   Gly Leu Pro Arg Tyr Val Ala Arg Leu
   1               5
(2) INFORMATION FOR SEQ ID NO:43:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:43:
   Gly Leu Pro Arg Tyr Val Val Cys Leu
   1               5
(2) INFORMATION FOR SEQ ID NO:44:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:44:
   Gly Leu Ser Arg Tyr Val Val Cys Leu
   1               5

## Description

### Government Support

The U.S. Government may have certain rights in this invention pursuant to grants awarded by the National Institutes of Health.

### Background of the Invention

Cytotoxic T lymphocytes (CTLs) play an essential role in fighting cells infected with viruses, intracellular bacteria and parasites, and tumor cells. They do so by direct cytotoxicity and by providing specific and nonspecific help to other immunocytes such as macrophages, B cells, and other T cells. Infected cells or tumor cells process antigen through intracellular events involving proteases. The processed antigen is presented on the cellular surface in the form of peptides bound to HLA class I molecules to T cell receptors on CTLs. MHC class I molecules can also bind exogenous peptides and present them to CTLs without intracellular processing.

At the present time it is difficult to accurately predict from the sequence of an antigenic protein how the protein will be processed and which peptide portions will bind HLA class I molecules and be presented to CTLs. Binding motifs have been predicted for some HLA class I molecules based on sequence analysis of peptides eluted from these molecules (Falk et al., Nature 351:290 (1991)). Further, of the peptides that are processed and do bind to HLA class I, which ones will contain CTL-recognizable epitopes is not yet predictable.

WO-A-94/03205 relates to a number of peptides which are capable of specifically binding motifs for MHC class I molecules and induce T cell activation. WO-A-94/20127 describes a number of HLA-A2.1 binding peptides that induce T cell activation. Both of the above documents were published after the filing date of the present application and are citeable under Art 54(3) only. WO-A-93/03753 relates to some peptides for use in defining epitopes that stimulate HLA-restricted cytotoxic T cell activity against hepatitis B virus antigens. Carbone et al, J. Exp. Med. 167 pp1767 - 1779 (1988) relates to a method and assay for detecting the induction of cytotoxic T cells by in vitro stimulation with peptides. No peptide disclosed in any of the above documents which is also claimed herein is entitled to an earlier priority date for the above documents than for the claims herein

Hepatitis B virus ("HBV") is a non-lytic virus which has currently infected approximately 250 million people worldwide. HBV infection in adults typically leads to an acute disease in the majority of cases, and to a chronic disease state in a minority of patients This ratio of acute to chronic is reversed when the infection occurs close to the time of birth. There is an increased incidence of hepatocellular carcinoma in chronic HBV infection. A small percentage of individuals who are infected with HBV in adulthood develop fulminant hepatitis associated with a strong immune response with high lethality.

While there is no effective treatment for HBV infection, vaccines have been developed in recent years to prevent HBV infection. The vaccines employ either HBV surface antigen (HBsAg) purified from the plasma of chronic HBV carriers, or HBsAg produced by recombinant DNA technology. Synthetic HBsAg peptide-based vaccines have also been proposed. See, for example, U.S. Patent Nos. 4,599,230 and 4,599,231. The anti-HBaAg vaccines, however, afford protection in only about 90% of immunized individuals. Those who are unimmunized, or immunized but unprotected, provide a significant reservoir of potential infection.

The contribution of CTLs to immunity to HBV antigens has been difficult to assess. Chisari et al. (Microbial Pathogen. 6:31 (1989)) have suggested that liver cell injury may be mediated by an HLA-Class I restricted, CD8⁺ cytotoxic T cell response to HBV encoded antigens. Class I major histocompatibility (MHC) -restricted cytotoxic T lymphocyte responses have been identified for a variety of other viruses, such as influenza. For example, Townsend et al., Cell 44:959 (1986) reported that epitopes of an influenza virus nucleoprotein recognized by cytotoxic T lymphocytes could be defined by synthetic peptides. In attempting to define the cytotoxic T lymphocyte response to HBV, it has been shown that peripheral blood lymphocytes from patients with acute and chronic HBV may be able to kill autologous hepatocytes in vitro, but the specificity of the cytolytic activity, its HLA restriction elements, and cellular phenotype were not established. See, Mondelli et al., J. Immunol. 129:2773 (1982) and Mondelli et al., Clin. Exp. Immunol. 6:311 (1987). Moriyama et al., Science 248:361-364 (1990), have reported that the HBV major envelope antigen is expressed at the hepatocyte surface in a form recognizable by envelope-specific antibodies and by MHC class I-restricted, CD8⁺ cytotoxic T lymphocytes.

As there is a large reservoir of individuals chronically infected with HBV, it would be desirable to stimulate the immune response of these individuals to respond to appropriate HBV antigens and thereby eliminate their infection. It would also be desirable to prevent the evolution to a chronic HBV infection in individuals suffering from an acute phase infection. Further, as the presently approved HBV vaccines do not elicit protective immunity in about 10% of immunized individuals, it would be desirable to elicit more effective immunity, such as by increasing or diversifying the immunogenicity of the vaccines. Quite surprisingly, the present invention fulfills these and other related needs.

### Summary of the Invention

The present invention provides peptides which induce MHC class I restricted cytotoxic T lymphocyte responses against HBV antigen. The peptides of interest are derived from the sequence of the HBV polymerase protein. According to a first aspect of the invention there is provided a CTL-inducing peptide comprising from eight to thirteen amino acids, wherein the CTL inducing peptide comprises The peptide may be optionally flanked and/or modified at one or both of the N- and C-termini, as desired.

In the various peptide embodiments it will be understood that the peptides can be polymerized, each to itself to form larger homopolymers, or with different peptides to form heteropolymers. In some instances peptides will be combined in a composition as an admixture and will not be linked. The peptide can also be conjugated to a lipid-containing molecules capable of enhancing a T lymphocyte response, or to a different peptide which induces a T-helper cell response, for example.

Compositions are described which comprise a peptide of the invention formulated with an additional peptide, a liposome, an adjuvant and/or a pharmaceutically acceptable carrier. Thus, pharmaceutical compositions can be used in methods of treating acute HBV infection, particularly in an effort to prevent the infection from progressing to a chronic or carrier state. Methods for treating chronic HBV infection and HBV carrier states are also described, where the pharmaceutical compositions are administered to infected individuals in amounts sufficient to stimulate immunogenically effective cytotoxic T cell responses against HBpol epitopes. For treating these infections it may be particularly desirable to combine the peptides which induce MHC class I restricted cytotoxic T lymphocyte responses against HBV antigen with other peptides or proteins that induce immune response to other HBV antigens, such as HBV envelope or core. To treat individuals with chronic or carrier state infections the compositions may be administered in repeated dosages over a prolonged period of time, as necessary, to resolve or substantially mitigate the infection and/or shedding of virus.

Vaccine compositions for preventing HBV infection, particularly chronic HBV infection, are also described. The vaccine compositions comprise an immunogenically effective amount of a HBV polymerase peptide mentioned above which induces a MHC class I restricted cytotoxic T lymphocyte response, such as HLA-A2, -A1, -A3, A-11, and/or -A24, and will typically further comprise an adjuvant, e.g., incomplete Freund's adjuvant or aluminum hydroxide. To achieve enhanced protection against HBV, the vaccine can further comprise components which elicit a protective antibody response to other HBV antigen, such as envelope (surface) antigen.

The invention may also be used in methods for diagnosis, where the peptides of the invention are used to determine the presence of lymphocytes in an individual which are capable of a cytotoxic T cell response to HBV polymerase antigen. The absence of such cells determines whether the individual of interest is susceptible to developing chronic HBV infection. Typically the lymphocytes are peripheral blood lymphocytes and the individual of interest is suffering from an acute HBV infection.

According to a second aspect of the invention there is provided a method for stimulating a cytotoxic T lymphocyte response to hepatitis B virus which comprises exposing cytotoxic T lymphocytes of a host to a peptide wherein the peptide is a peptide comprising from eight to thirteen amino acids, wherein the CTL-inducing peptide comprises

In a further aspect there is provided a method for stimulating a cytotoxic T lymphocyte response to hepatitis B virus which comprises exposing cytotoxic T lymphocytes of a host to a peptide which contains a CTL epitope and which comprises from eight to thirteen amino acids, wherein the CTL-inducing peptide is selected from a peptide comprising and wherein the host cells exposed to the CTL-inducing peptides are HLA-A2 restricted, and wherein the cytotoxic T cells are removed from the host prior to being exposed to the CTL-inducing peptide.

In another aspect there is provided use of a CTL-inducing peptide as described in the above paragraph for the preparation of a medicament for use in a method for stimulating a cytotoxic T lymphocyte response to hepatitis B virus, which method comprises:(i) exposing cytotoxic T lymphocytes of a host to the peptide, wherein the host cells exposed to the CTL-inducing peptides are HLA-A2 restricted, and wherein the cytotoxic T cells are removed from the host prior to being exposed to the CTL-inducing peptide, and (ii) returning the stimulated CTLs to the host.

### Brief Description of the Drawings

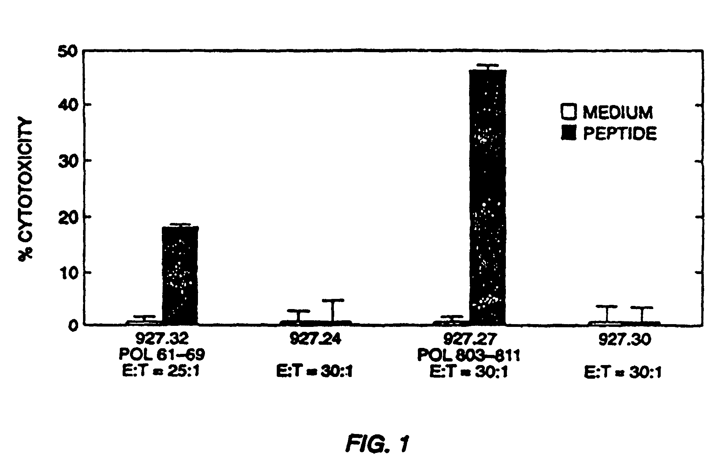

Fig. 1 shows the CTL response to two polymerase peptides that contain the HLA-A2 motif in a patient using target cells pulsed with peptide that match only at HLA-A2.

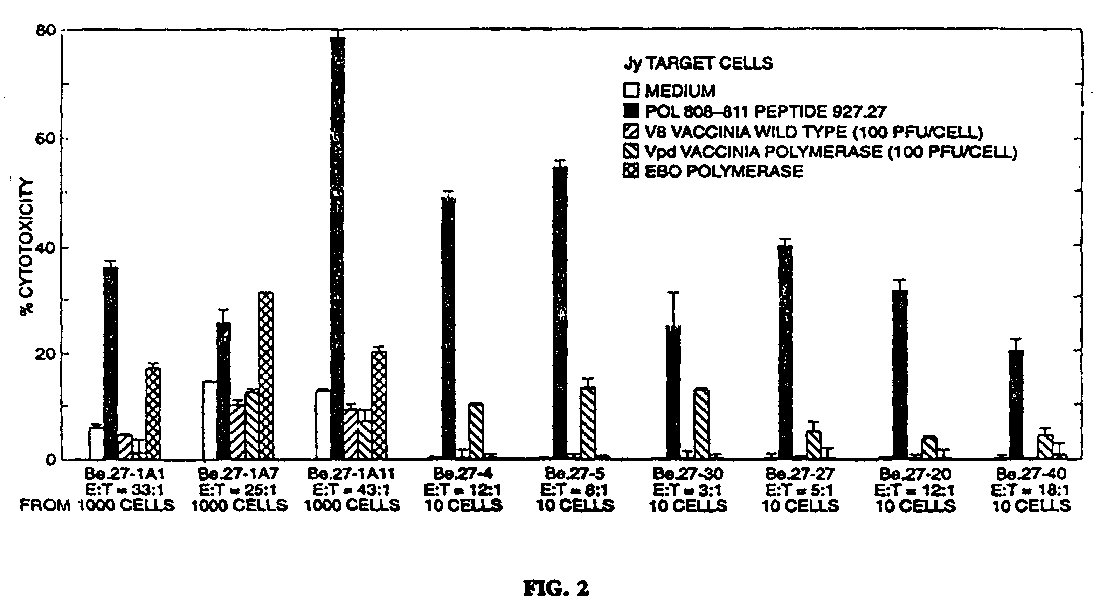

Fig. 2 shows the ability of several polymerase 803-811 peptide specific clones to recognize endogenously synthesized polymerase.

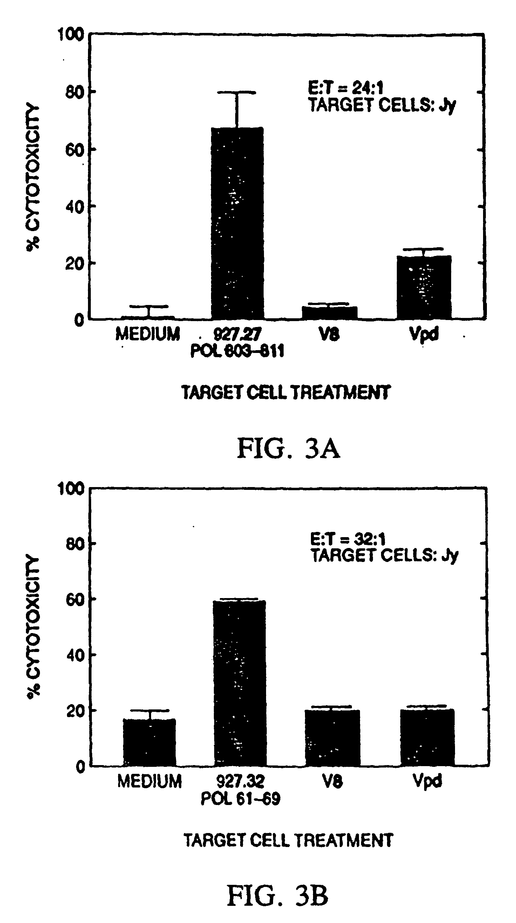

Fig. 3 shows that the CTL response to polymerase peptide 803-811 can recognize cells pulsed with peptide and endogenously synthesized polymerase (Vpo1), whereas the CTL response to polymerase peptide 61-69 only recognized cells pulsed with the 61-69 peptide.

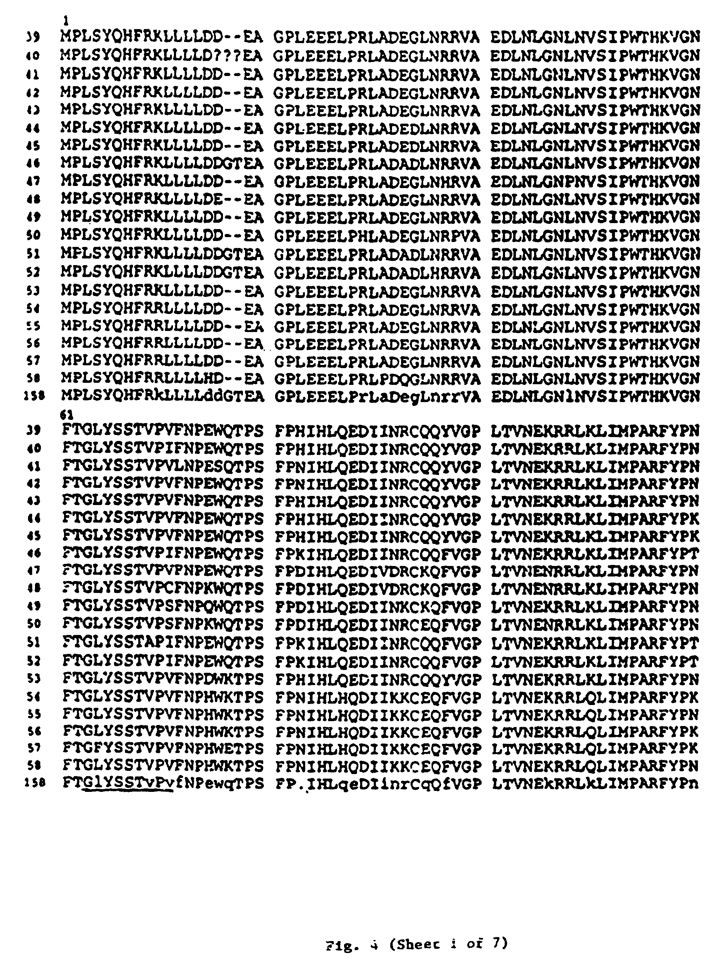

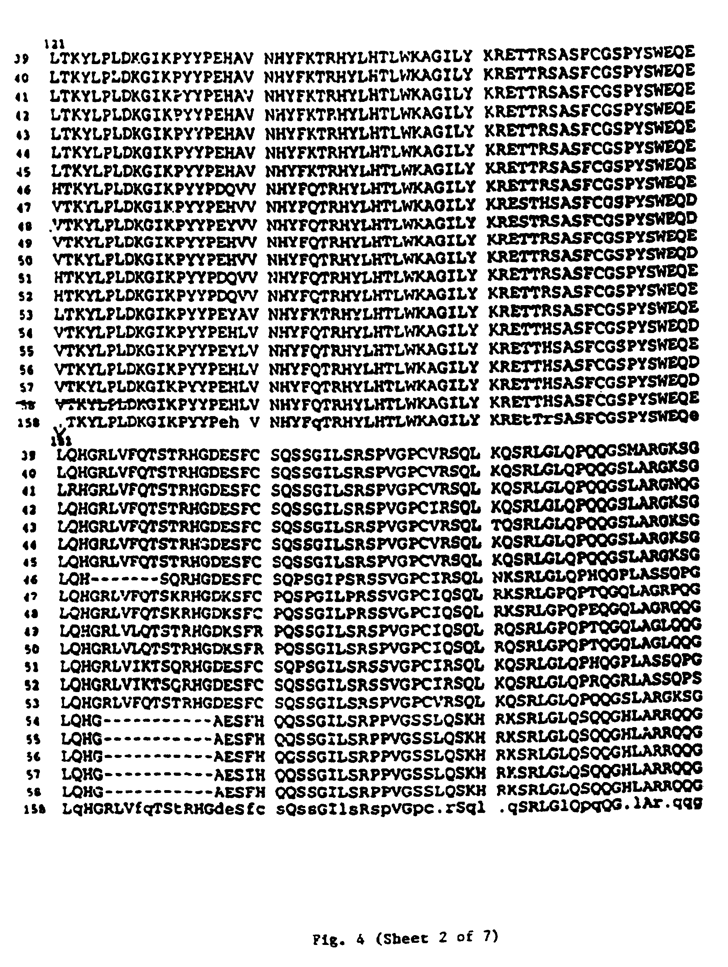

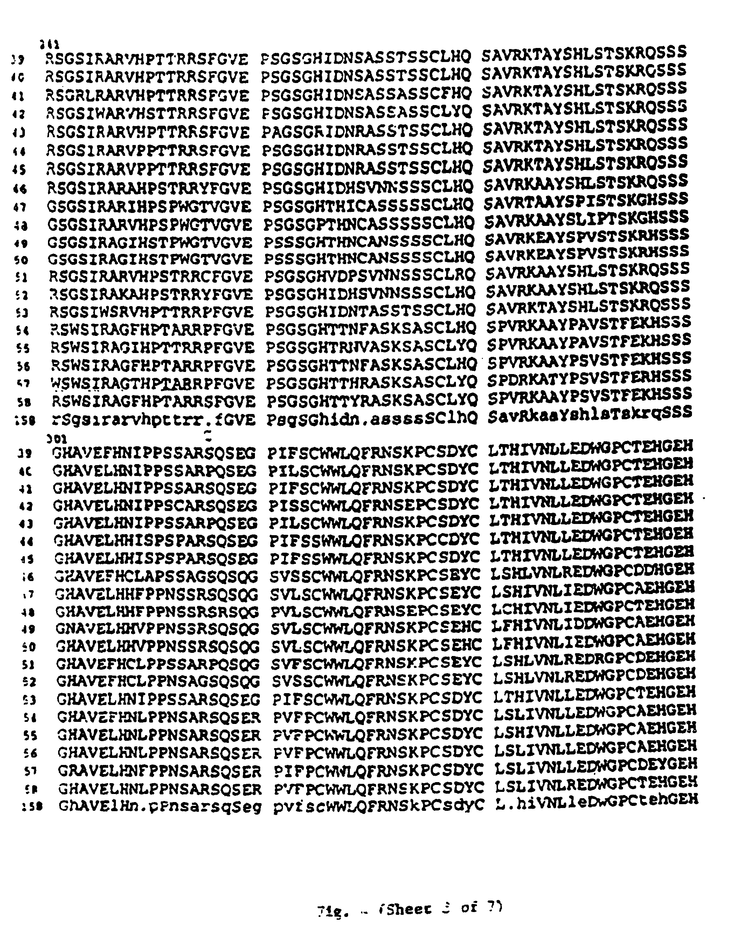

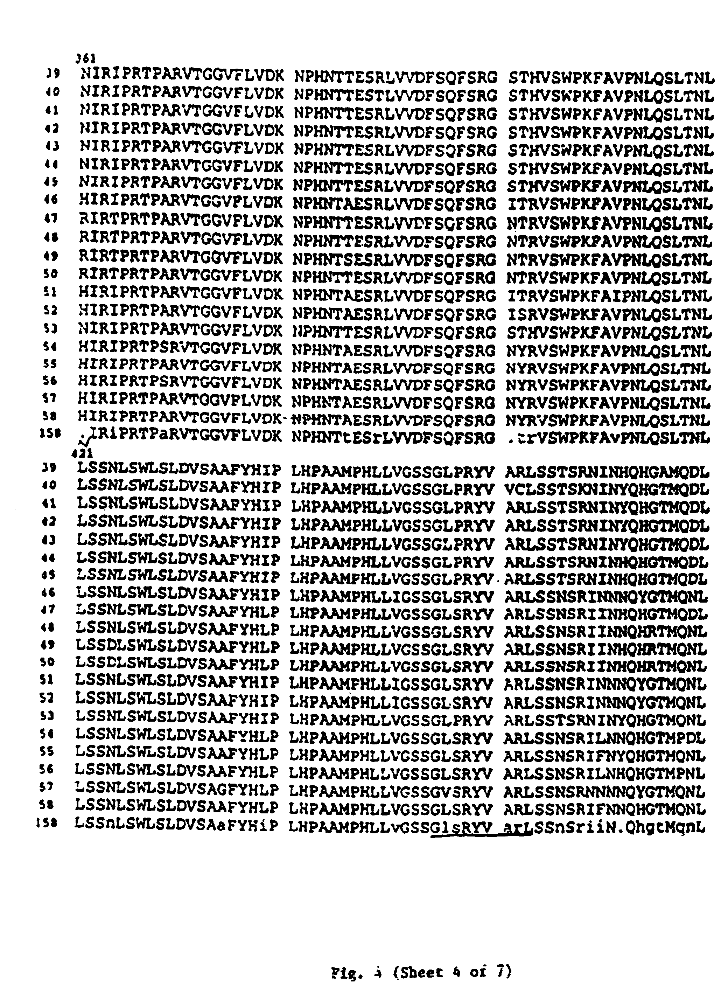

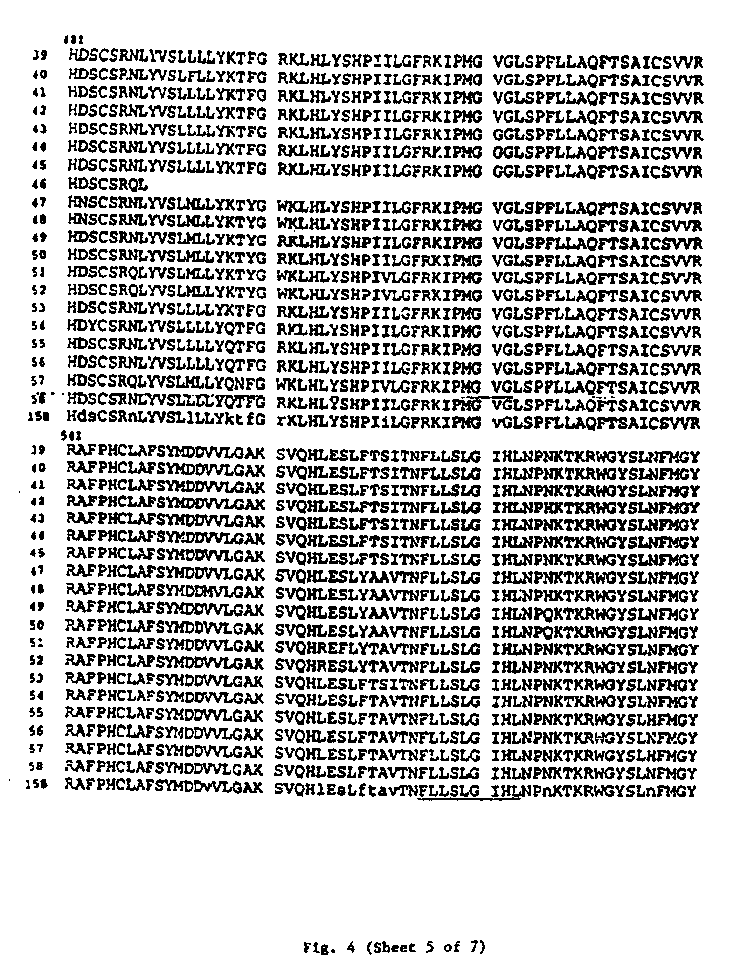

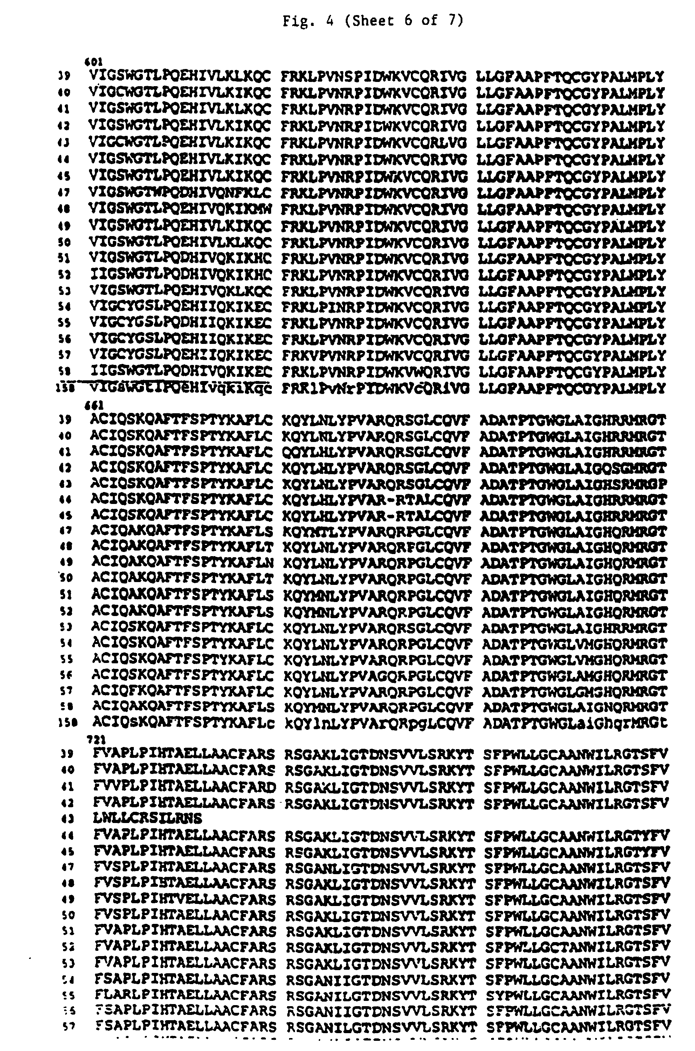

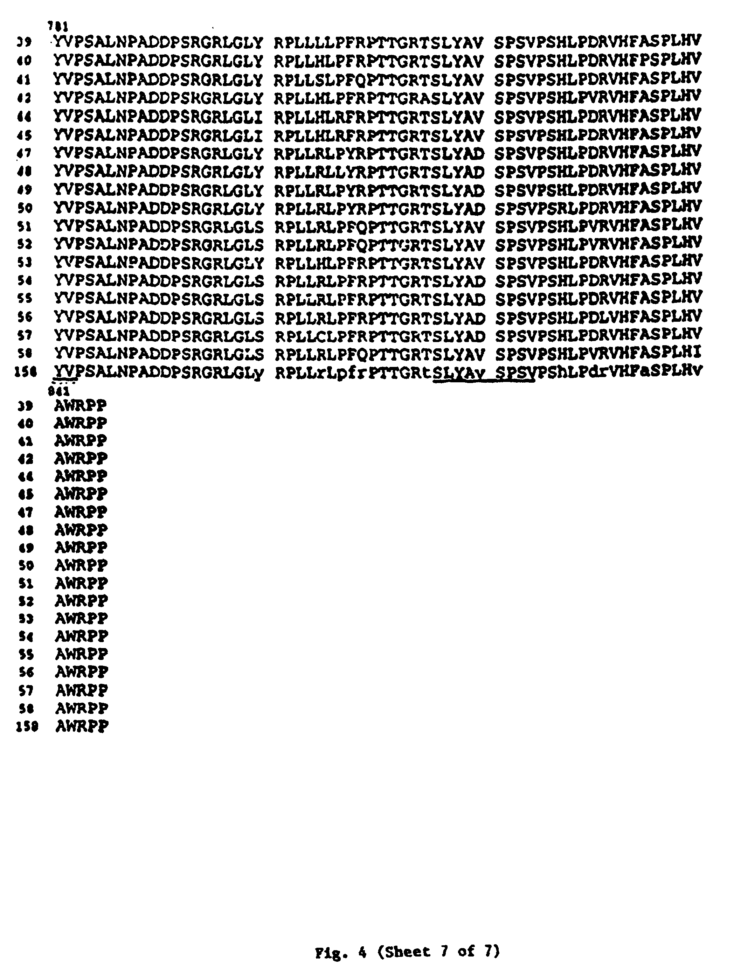

Fig. 4 shows the aligned amino acid sequences of 20 cloned HBV polymerase proteins; line 158 is a consensus sequence where capital letters represent 100% consensus, lower case letters represent >50% consensus, and "." is <50% consensus.

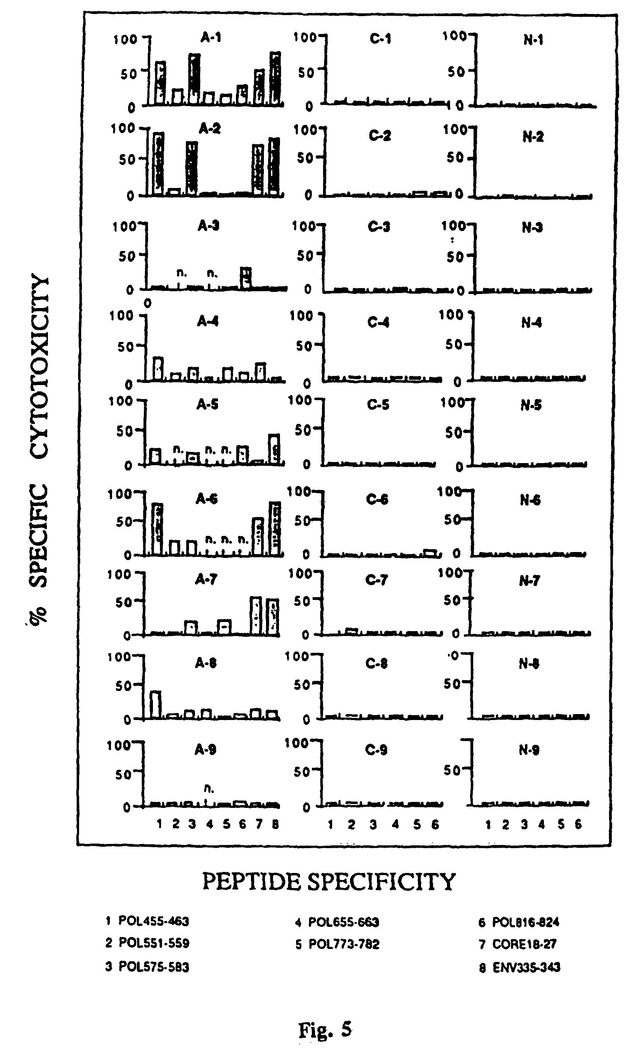

Fig. 5 shows HBV specific CTL response in patients with acute hepatitis (A-1 - A-9), chronic hepatitis (C-1 - C-9) and normal subjects (N-1 - N-9). PBMC were stimulated with the peptides indicated for 2 weeks and tested in a 4-h ⁵¹Cr-release assay against JY target cells prepulsed overnight with the same peptide. Peptide-specific cytotoxicity was measured by subtracting the ⁵¹Cr-release by JY target cells not prepulsed with the peptide from the ⁵¹Cr-release by JY target cells prepulsed with the peptide. Results shown represent percent specific lysis in a 4 hr ⁵¹Cr-release assay at an E:T of 50:1.

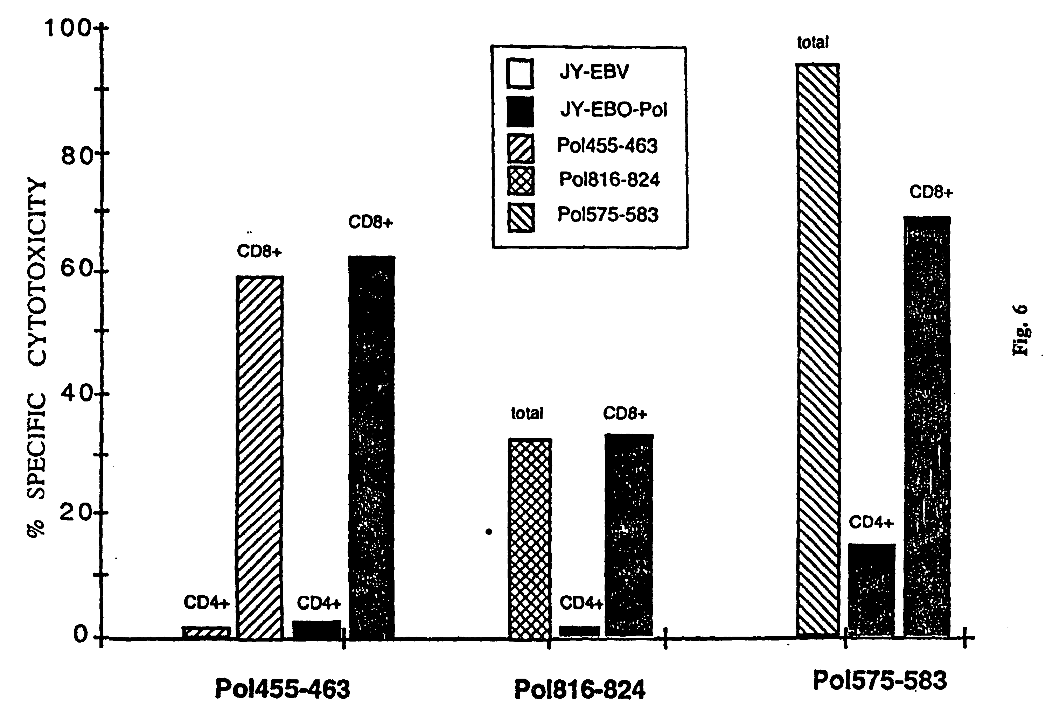

Fig. 6 shows CD8+ cells recognize endogenously synthesized antigen in target cells sharing the HLA-A2 allele (Patient A-1). Epitope-specific lines were generated by stimulating PBMC with the individual peptide for three weeks with weekly restimulation. On day 15 of CD4+ (positive selection) and CD8+ (negative selection) enriched lines were generated from the original bulk culture by panning. FACS-analysis showed an average enrichment by a factor of 3. Results shown represent percent specific lysis in a 4 hr ⁵¹Cr-release assay at an E:T of 30:1. Targets (JY-EBV) were either pulsed with the corresponding peptide overnight stably transfected with the polymerase expression vector.

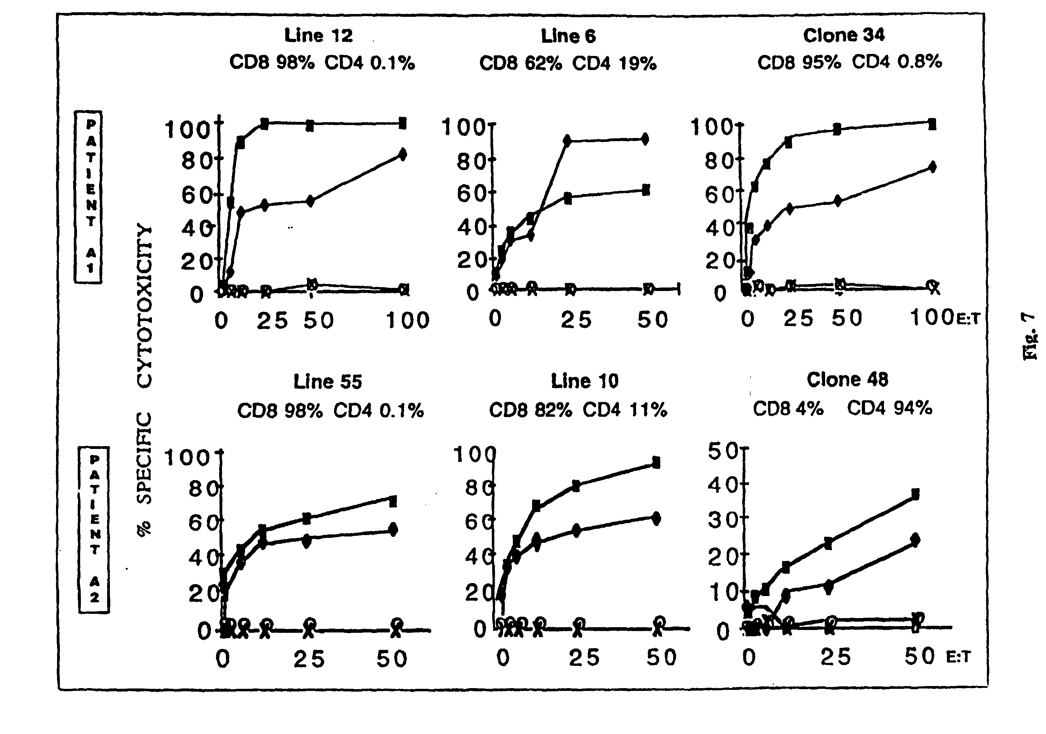

Fig. 7 shows CTL-response to Pol455-463 GLSRYVARL [Seq ID No. 2]. Epitope-specific lines and clones, generated by stimulation with Pol455-463 peptide, were tested at varying E:T ratios against targets cells (JY-EBV), pulsed with the corresponding peptide ( 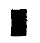 ) overnight or infected with recombinant vaccinia virus that express the HBV polymerase polypeptide (◆), in a standard 4 hr ⁵¹Cr-release assay. Wild-type vaccinia virus (Wt) (X) or JY-EBV peptides without peptide (○) were used as a control.

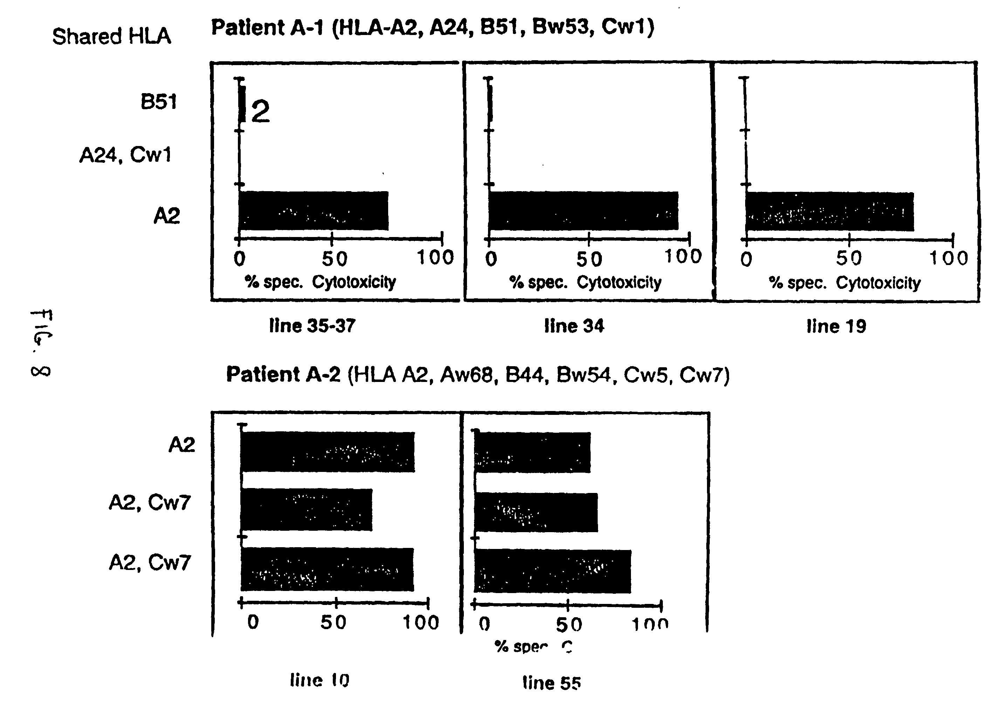

Fig. 8 shows HLA-restriction of epitope Pol455-463. Pol455-463-specific lines from patient A-1 and A-2, generated by stimulation with Pol455-463 peptide, were tested against allogeneic partly HLA-matched EBV-B cells prepulsed overnight with 10 µg/ml of the same peptide. Sharing HLA class I at other loci did not render target cells susceptible to lysis. Cytotoxicity was measured at E:T of 50:1 in a 4 hr ⁵¹Cr-release assay.

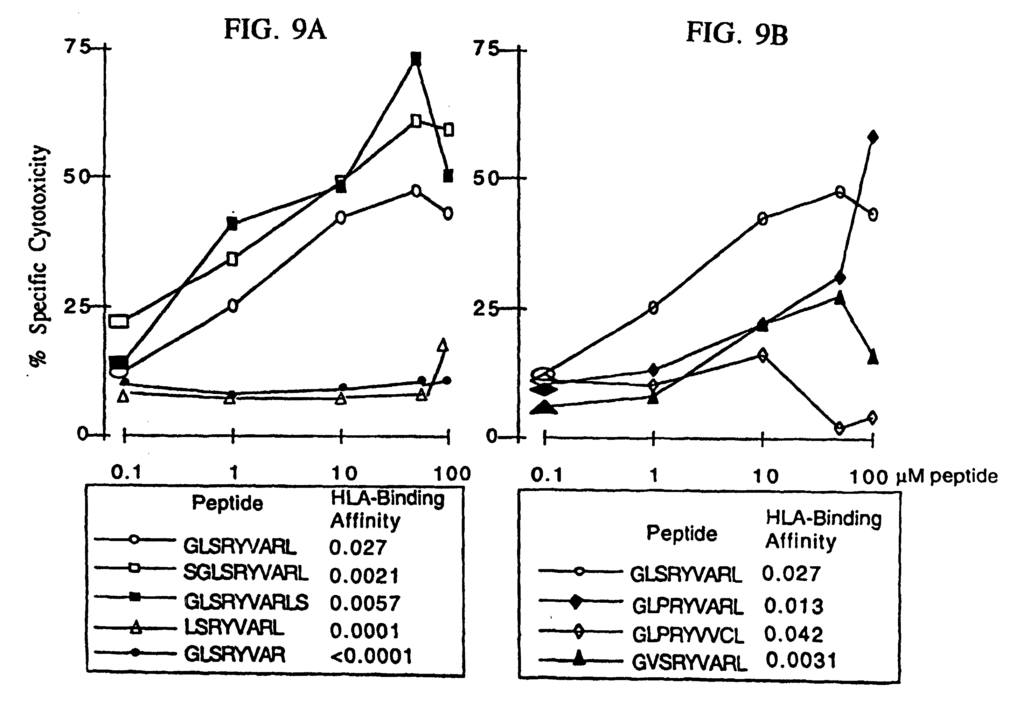

Fig. 9 shows recognition of truncated, elongated (a) or variant peptides (b) by Pol455-463 specific CTL-lines, generated by weekly stimulation of PBMC from patient A-1 with peptide Pol455-463 for 4 weeks. Cytotoxicity was measured at E:T of 50:1 in a 4 hr ⁵¹Cr-release assay against JY-EBV cells prepulsed with varying amounts of the same peptide overnight.

### Description of the Specific Embodiments

The present invention provides peptides derived from HBV polymerase proteins for use in compositions and methods for the treatment, prevention and diagnosis of HBV infection. The peptides stimulate MHC HLA-class I restricted cytotoxic T lymphocyte responses against HBV infected cells. The stimulated cytotoxic T lymphocytes are able to kill the infected cells or inhibit viral replication and thus interrupt or substantially prevent infection, including chronic HBV infection. A peptide effective in eliciting a cytotoxic T cell response may also be combined with an immunogen capable of eliciting a T-helper response.

The peptides employed in the invention are derived from the sequence of the HBV polymerase protein (HBpol), particularly CTL epitopes within HBpol 455-463, HBpol773-782, or HBpol816-824, where the numbering is according to Galibert et al., supra.

By HBV cytotoxic T lymphocyte inducing "peptide" or "oligopeptide" of the present invention is meant a chain of at least four HBV amino acid sequence residues, preferably at least six, more preferably eight or nine; sometimes ten to twelve residues, and usually fewer than about fifty residues, more usually fewer than about thirty-five, and preferably fewer than twenty-five, e.g., eight to seventeen amino acid residues derived from an HBc sequence. It may be desirable to optimize peptides of the invention to a length of eight to twelve amino acid residues, more preferably nine to eleven, commensurate in size with endogenously processed viral peptides that are bound to MHC class I molecules on the cell surface. See generally, Schumacher et al., Nature 350:703-706 (1991); Van Bleek et al., Nature 348:213-216 (1990); Rotzschke et al., Nature 348:252-254 (1990); and Falk et al., Nature 351:290-296 (1991), which are incorporated herein by reference. As set forth in more detail below, usually the peptides will have at least a majority of amino acids which are homologous to a corresponding portion of contiguous residues of the HBV pol sequences herein, and contain a CTL-inducing epitope.

The peptides can be prepared "synthetically," as described hereinbelow, or by recombinant DNA technology. Although the peptide will preferably be substantially free of other naturally occurring HBV proteins and fragments thereof, in some embodiments the peptides can be synthetically conjugated to native fragments or particles. The term peptide is used interchangeably with polypeptide in the present specification to designate a series of amino acids connected one to the other by peptide bonds between the alpha-amino and alpha-carboxy groups of adjacent amino acids. The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. By biological activity is meant the ability to bind an appropriate MHC molecule and induce a cytotoxic T lymphocyte response against HBV antigen or antigen mimetic. By a cytotoxic T lymphocyte response is meant a CD8⁺ T lymphocyte response specific for an HBV antigen of interest, wherein CD8⁺, MHC class I-restricted T lymphocytes are activated. The activated T lymphocytes secrete lymphokines (e.g., gamma interferon) liberate products (e.g., serine esterases) that inhibit viral replication in infected autologous cells or transfected cells, with or without cell killing.

The terms "homologous", "substantially homologous", and "substantial homology" as used herein denote a sequence of amino acids having at least 50% identity wherein one sequence is compared to a reference sequence of amino acids. The percentage of sequence identity or homology is calculated by comparing one to another when aligned to corresponding portions of the reference sequence.

Described herein are peptides which contain CTL-inducing epitopes derived from various epitopic regions of the HBV polymerase protein. The peptides are from the region of HBpol₆₁₋₆₉ and include peptides derived from those sequence regions which contain one or more CTL-inducing HLA class I-restricted epitopic site(s) of at least seven contiguous amino acids. A majority of the amino acids of the peptide will be identical or substantially homologous the amino acids of the corresponding portions of the naturally occurring HBpol₆₁₋₆₉ sequence, where HBpol₆₁₋₆₉ has the following sequence (for HBV subtype ayw): The peptides of this HBpol₆₁₋₆₉ region and the polymerase peptide regions of the invention can be optionally flanked and/or modified at one or both of the N- and C-termini, as desired, by amino acids from HBV sequences, including HBpol, amino acids added to facilitate linking, other N- and C-terminal modifications, linked to carriers, etc., as further described herein. The peptide HBpol61-69 induces a cytotoxic T lymphocyte response which is mediated by at least the MHC class I molecule HLA-A2.

HBpol region peptides containing CTL epitopes of the invention comprise the peptide HBpol 455-463, and peptides derived from HBpol455-463 which contain a CTL-inducing HLA class I-restricted epitopic site(s) of at least seven contiguous amino acids. A majority of the amino acids of the peptide will be identical to the amino acids of the corresponding portions of the naturally occurring HBpol455-463 sequence, where HBpol 455-463 has the sequence (for HBV subtype ayw): wherein the selected peptide can be flanked and/or modified at one or both termini as described herein. The peptide HBpol 455-463 induces a cytotoxic T lymphocyte response which is mediated by at least the MHC class I molecule HLA-A2.

Yet other HBpol region peptides containing CTL epitopes used in embodiments of the invention comprise the peptide HBpol 773-782, and peptides derived from HBpol773-782 which contain a CTL-inducing HLA class I-restricted epitopic site(s) of at least seven contiguous amino acids. A majority of the amino acids of the peptide will be identical to the amino acids of the corresponding portions of the naturally occurring HBpol773-782 sequence, where HBpol 773-782 has the sequence (for HBV subtype ayw): wherein the selected peptide can be flanked and/or modified at one or both termini as described herein. The peptide HBpol 773-782 induces a cytotoxic T lymphocyte response which is mediated by at least the MHC class I molecule HLA-A2. Other HBpol peptide embodiments of the invention are prepared from the region of HBpol803-811. Peptides derived from this region contain at least one CTL-inducing HLA class I-restricted epitopic site, and will typically be at least seven amino acids, more usually nine, ten or eleven amino acids or more. A majority of the amino acids of the peptide will be identical or substantially homologous to the amino acids of the corresponding portions of the naturally occurring HBpol803-811 sequence, where HBpol803-811 has the sequence (for HBV subtype ayw): wherein the selected peptide can be flanked and/or modified at one or both termini as described herein. The peptide HBpol 803-811 induces a cytotoxic T lymphocyte response which is mediated by at least the MHC class I molecule HLA-A2.

Other HBpol peptide used in embodiments of the invention are prepared from the region of HBpol816-824. Peptides derived from this region contain at least one CTL-inducing HLA class I-restricted epitopic site, and will typically be at least seven amino acids, more usually nine, ten or eleven amino acids or more. A majority of the amino acids of the peptide will be identical to the amino acids of the corresponding portions of the naturally occurring HBpol816-824 sequence, where HBpol816-824 has the sequence (for HBV subtype ayw): wherein the selected peptide can be flanked and/or modified at one or both termini as described herein. The peptide HBpol 816-824 induces a cytotoxic T lymphocyte response which is mediated by at least the MHC class I molecule HLA-A2.

Other HBpol peptides described herein are prepared from the regions of HBpol4-13, HBpol108-116, HBpol139-147, HBpol151-160, HBpol152-161, HBpol505-514, HBpol551-559, HBpol575-583, HBpol655-663, HBpol748-757, or HBpol758-766. A peptide prepared from one of the aforementioned regions contains at least one CTL-inducing HLA class I-restricted epitopic site, and will typically be at least seven amino acids, more usually nine, ten or eleven amino acids or more. A majority of the amino acids of the peptide will be identical or substantially homologous to the amino acids of the corresponding portions of the naturally occurring HBpol sequence, where the HBpol regions have the sequences (for HBV subtype ayw): wherein the selected peptide can be flanked and/or modified at one or both termini as described herein. The peptide HBpol151-160 induces a CTL response which is mediated by at least the MHC class I molecule HLA-A1. The peptides HBpol551-559 and HBpol655-663 induce a CTL response which is mediated by at least the MHC class I molecule HLA-A2. The peptide HBpol575-583 induces a CTL response which is mediated by at least the MHC class I molecule HLA-A2.1. The peptides HBpol108-116, HBpol139-147, HBpol152-161, and HBpol748-757 induce a CTL response which is mediated by at least the MHC class I molecule HLA-A3 (HBpol748-757 appearing to also be restricted by A24). The peptides HBpol4-13, HBpol505-514, and HBpol758-766 induce CTL responses which are mediated by at least the MHC class I molecule HLA-A24.

As mentioned above, additional amino acids can be added to the termini of an oligopeptide or peptide to provide for ease of linking peptides one to another, for coupling to a carrier, support or a larger peptide, for reasons discussed herein, or for modifying the physical or chemical properties of the peptide or oligopeptide, and the like. Amino acids such as tyrosine, cysteine, lysine, glutamic or aspartic acid, and the like, can be introduced at the C- or N-terminus of the peptide or oligopeptide. In addition, the peptide or oligopeptide sequences can differ from the natural sequence by being modified by terminal-NH₂ acylation, e.g., acetylation, or thioglycolic acid amidation, terminal-carboxy amidation, e.g., ammonia, methylamine, etc. In some instances these modifications may provide sites for linking to a support or other molecule.

It will be understood that the HBV peptides of the present invention or analogs or homologs thereof which have cytotoxic T lymphocyte stimulating activity may be modified as necessary to provide certain other desired attributes, e.g., improved pharmacological characteristics, while increasing or at least retaining substantially the biological activity of the unmodified peptide. For instance, the peptides can be modified by extending, decreasing or substituting amino acids in the peptide sequence by, e.g., the addition or deletion of amino acids on either the amino terminal or carboxy terminal end, or both, of peptides derived from the sequences disclosed herein. The peptides may be modified to substantially enhance the CTL inducing activity, such that the modified peptide analogs have CTL activity greater than a peptide of the wild-type sequence. For example, it may be desirable to increase the hydrophobicity of the N-terminal of a peptide, particularly where the second residue of the N-terminal is hydrophobic and is implicated in binding to the HLA restriction molecule. By increasing hydrophobicity at the N-terminal, the efficiency of the presentation to T cells may be increased. Peptides prepared from other disease associated antigens, particularly those containing CTL inducing epitopes for which a host may not have significant CTL activity, may be made CTL-inducing by substituting hydrophobic residues at the N-terminus of the peptide where the second residue is normally hydrophobic.

The peptides employed in the subject invention need not be identical to peptides HBpol 455-463 (Gly-Leu-Ser-Arg-Tyr-Val-Ala-Arg-Leu) [Seq ID No. 2]; HBpol773-782 (Ile-Leu-Arg-Gly-Thr-Ser-Phe-Val-Tyr-Val) (Seq ID No. 3]; or HBpol816-824 (Phe-Leu-Leu-Ser-Leu-Gly-Ile-His-Leu) [Seq ID No. 5], so long as the subject compounds are able to provide for cytotoxic T lymphocytic activity against at least one of the four major subtypes of HBV. Although different strains of HBV exist, they each share at least one common envelope determinant, which is designated "a". Each strain also has two other envelope determinants, one of which is either "d" or "y", and the second is either "w" or "r". Thus, there are four possible subtypes of the virus: adw, ayw, adr, and ayr. The cloning, sequencing and expression of HBV are described in GB 2034323, EP 13828, U.S. 4,935,235, and the complete sequence of the HBV envelope region is also described in Galibert et al., Nature 281:646 (1979). Amino acid sequences are described in the GenBank-72 database for 20 different HBV strains, including 7 of the adw subtype, 5 of the ayw subtype, 7 of the adr subtype, and 1 strain of the ayr subtype.

Therefore, the peptides may be subject to various changes, such as insertions, deletions, and substitutions, either conservative or non-conservative, where such changes provide for certain advantages in their use. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu; Asp, Glu; Asn, Gln; Ser, Thr; Lys, Arg; and Phe, Tyr. Usually, the portion of the sequence which is intended to substantially mimic an HBV cytotoxic T lymphocyte stimulating epitope will not differ by more than about 20% from the sequence of at least one subtype of HBV, except where additional amino acids may be added at either terminus for the purpose of modifying the physical or chemical properties of the peptide for, e.g., ease of linking or coupling, and the like. Where regions of the peptide sequences are found to be polymorphic among HBV subtypes, it may be desirable to vary one or more particular amino acids to more effectively mimic differing cytotoxic T-lymphocyte epitopes of different HBV strains or subtypes.

Within the peptide sequences described herein, including the representative peptides listed above, there are residues (or those which are substantially functionally equivalent) which allow the peptide to retain their biological activity, i.e., the ability to stimulate a class I-restricted cytotoxic T-lymphocytic response against HBV infected cells or cells which express HBV antigen. These residues can be identified by single amino acid substitutions, deletions, or insertions. In addition, the contributions made by the side chains of the residues can be probed via a systematic scan with a specified amino acid (e.g., Ala). Peptides which tolerate multiple substitutions generally incorporate such substitutions as small, relatively neutral molecules, e.g., Ala, Gly, Pro, or similar residues. The number and types of residues which can be substituted, added or subtracted will depend on the spacing necessary between the essential epitopic points and certain conformational and functional attributes which are sought (e.g., hydrophobicity vs. hydrophilicity). If desired, increased binding affinity of peptide analogues to its MHC molecule for presentation to a cytotoxic T-lymphocyte can also be achieved by such alterations. Generally, any spacer substitutions, additions or deletions between epitopic and/or conformationally important residues will employ amino acids or moieties chosen to avoid steric and charge interference which might disrupt binding.

Peptides which tolerate multiple substitutions while retaining the desired biological activity may also be synthesized as D-amino acid containing peptides. Such peptide may be synthesized as "inverso" or "retro-inverso" forms, that is, by replacing L-amino acids of a sequence with D-amino acids, or by reversing the sequence of the amino acids and replacing the L-amino acids with D-amino acids. As the D-peptides are substantially more resistant to peptidases, and therefore are more stable in serum and tissues compared to their L-peptide counterparts, the stability of D-peptides under physiological conditions may more than compensate for a difference in affinity compared to the corresponding L-peptide. Further, L-amino acid-containing peptides with or without substitutions can be capped with a D-amino acid to inhibit exopeptidase destruction of the antigenic peptide.

In addition to the exemplary peptides described herein, described are methods for identifying other epitopic regions associated with said peptide regions capable of inducing MHC-restricted cytotoxic T lymphocyte responses against HBV. The methods comprise obtaining peripheral blood lymphocytes (PBL) from infected or uninfected individuals and exposing (stimulating) the cells with synthetic peptide or polypeptide fragments derived from a peptide'region of HBpol4-13 (Ser-Tyr-Gln-His-Phe-Arg-Lys-Leu-Leu-Leu) [Seq ID No. 12]; HBpol61-69 (Gly-Leu-Tyr-Ser-Ser-Thr-Val-Pro-Val) [Seq ID No. 1]; HBpol108-116 (Arg-Leu-Lys-Leu-Ile-Met-Pro-Ala-Arg) (Seq ID No. 13]; HBpol139-147 (Val-Val-Asn-His-Tyr-Phe-Gln-Thr-Arg) [Seq ID No. 14]; HBpol151-160 (His-Thr-Leu-Trp-Lys-Ala-Gly-Ile-Leu-Tyr) [Seq ID No. 15]; HBpol152-161 (Thr-Leu-Trp-Lys-Ala-Gly-Ile-Leu-Tyr-Lys) [Seq ID No. 16]; HBpol 455-463 (Gly-Leu-Ser-Arg-Tyr-Val-Ala-Arg-Leu) [Seq ID No. 2]; HBpol505-514 (Leu-Tyr-Ser-His-Pro-Ile-Ile-Leu-Gly-Phe) (Seq ID No. 17); HBpol551-559 (Tyr-Met-Asp-Asp-Val-Val-Leu-Gly-Ala) [Seq ID No. 18]; HBpol575-583 (Phe-Leu-Leu-Ser-Leu-Gly-Ile-His-Leu) [Seq ID No. 19]; HBpol655-663 (Ala-Leu-Met-Pro-Leu-Tyr-Ala-Cys-Ile) [Seq ID No. 20]; HBpol748-757 (Gly-Thr-Asp-Asn-Ser-Val-Val-Leu-Ser-Arg) [Seq ID No. 21]; HBpol758-766 (Lys-Tyr-Thr-Ser-Phe-Pro-Trp-Leu-Leu) [Seq ID No. 22]; HBpol773-782 (Ile-Leu-Arg-Gly-Thr-Ser-Phe-Val-Tyr-Val) [Seq ID No. 3]; HBpol803-811 (Ser-Leu-Tyr-Ala-Asp-Ser-Pro-Ser-Val) [Seq ID No. 4]; or HBpol816-824 (Phe-Leu-Leu-Ser-Leu-Gly-Ile-His-Leu) [Seq ID No. 5]. Pools of overlapping synthetic peptides, each typically about 8 to 20 residues long, preferably 9-12 residues, can be used to stimulate the cells. Active peptides can be selected from pools which induce cytotoxic T lymphocyte activity. The ability of the peptides to induce specific cytotoxic activity is determined by incubating the stimulated PBL with autologous labeled (e.g., ⁵¹Cr) target cells (such as HLA matched macrophages, T cells, fibroblasts or B lymphoblastoid cells) infected or transfected with the HBV subgenomic fragments thereof, such that the targeted antigen is synthesized endogenously by the cell (or the cell is pulsed with the peptide of interest), and measuring specific release of label.

Once a peptide having an epitopic region which stimulates a cytotoxic T lymphocyte response is identified, the MHC restriction element of the response can be determined. This involves incubating the stimulated PBL or short term lines thereof with a panel of (labeled) target cells of known HLA types which have been pulsed with the peptide of interest, or appropriate controls. The HLA allele(s) of cells in the panel which are lysed by the CTL are compared to cells not lysed, and the HLA restriction element(s) for the cytotoxic T lymphocyte response to the antigen of interest is identified.

Carbone et al., J. Exp. Med. 167:1767 (1988), have reported that stimulation with peptides may induce cytotoxic T lymphocytes with low affinity for corresponding endogenous protein, such that repetitive peptide stimulation may yield cytotoxic T lymphocytes that recognize peptide but not native antigen. As the inability of stimulated cytotoxic T lymphocytes to recognize native HBV proteins would be undesirable in the development of HBV peptide therapeutics and vaccine compositions, methods to circumvent this potential limitation are used. A sequential restimulation of cytotoxic T cells is employed in the present invention to identify and select T cells with a higher affinity for naturally processed antigen than for a synthetic peptide. Short term cytotoxic T lymphocyte lines are established by restimulating activated PBL. Cells stimulated with peptide are restimulated with peptide and recombinant or native HBV antigen, e.g., HBpol. Cells having activity are also stimulated with an appropriate T cell mitogen, e.g., phytohemagglutinin (PHA). The restimulated cells are provided with irradiated allogeneic PBLs as an antigen nonspecific source of T cell help, and HBV antigen. To selectively expand the population of cytotoxic T lymphocytes that recognize native HBV antigen and to establish long term lines, PBL from a patient are first stimulated with peptide and recombinant or native HBV antigen, followed by restimulation with HLA-matched B lymphoblastoid cells that stably express the corresponding HBV antigen polypeptide. The cell lines are re-confirmed for the ability to recognize endogenously synthesized antigen using autologous and allogeneic B-lymphoblastoid or other cells transfected or infected with appropriate antigen.

Having identified different peptides which contribute to inducing anti-HBV cytotoxic T lymphocyte responses in one or more patients or HLA types, in some instances it may be desirable to join two or more peptides in a composition. The peptides in the composition can be identical or different, and together they should provide equivalent or greater biological activity than the parent peptide(s). For example, using the methods described herein, two or more peptides may define different or overlapping cytotoxic T lymphocyte epitopes from a particular region, e.g., the HBpol4-13 (Ser-Tyr-Gln-His-Phe-Arg-Lys-Leu-Leu-Leu) (Seq ID No. 12]; HBpol61-69 (Gly-Leu-Tyr-Ser-Ser-Thr-Val-Pro-Val) [Seq ID No. 1]; HBpol108-116 (Arg-Leu-Lys-Leu-Ile-Met-Pro-Ala-Arg) [Seq ID No. 13]; HBpol139-147 (Val-Val-Asn-His-Tyr-Phe-Gln-Thr-Arg) (Seq ID No. 14]; HBpol151-160 (His-Thr-Leu-Trp-Lys-Ala-Gly-Ile-Leu-Tyr) [Seq ID No. 15); HBpol152-161 (Thr-Leu-Trp-Lys-Ala-Gly-Ile-Leu-Tyr-Lys) (Seq ID No. 16]; HBpol 455-463 (Gly-Leu-Ser-Arg-Tyr-Val-Ala-Arg-Leu) (Seq ID No. 2]; HBpol505-514 (Leu-Tyr-Ser-His-Pro-Ile-Ile-Leu-Gly-Phe) [Seq ID No. 17]; HBpol551-559 (Tyr-Met-Asp-Asp-Val-Val-Leu-Gly-Ala) [Seq ID No. 18); HBpol575-583 (Phe-Leu-Leu-Ser-Leu-Gly-Ile-His-Leu) (Seq ID No. 19); HBpol655-663 (Ala-Leu-Met-Pro-Leu-Tyr-Ala-Cys-Ile) (Seq ID No. 20); HBpol748-757 (Gly-Thr-Asp-Asn-Ser-Val-Val-Leu-Ser-Arg) [Seq ID No. 21]; HBpol758-766 (Lys-Tyr-Thr-Ser-Phe-Pro-Trp-Leu-Leu) [Seq ID No. 22]; HBpol773-782 (Ile-Leu-Arg-Gly-Thr-Ser-Phe-Val-Tyr-Val) (Seq ID No. 3); HBpol803-811 (Ser-Leu-Tyr-Ala-Asp-Ser-Pro-Ser-Val) [Seq ID No. 4); or HBpol816-824 (Phe-Leu-Leu-Ser-Leu-Gly-Ile-His-Leu) (Seq ID No. 5] peptides, which peptides can be combined in a "cocktail" to provide enhanced immunogenicity for cytotoxic T lymphocyte responses. Moreover, peptides of one region can be combined with peptides of other HBV regions, from the same or different HBV protein, particularly when a second or subsequent peptide has a MHC restriction element different from the first. Other CTL-inducing HBV peptides are described in co-pending application USSN WO94/19011. This composition of peptides can be used to effectively broaden the immunological coverage provided by therapeutic, vaccine or diagnostic methods and compositions of the invention among a diverse population. For example, the different frequencies of HLA alleles among prevalent ethnic groups (caucasian, asian and african blacks) are shown in Table I below. Therapeutic or vaccine compositions of the invention may be formulated to provide potential therapy or immunity to as high a percentage of a population as possible.

**TABLE I.**

| HLA ALLELE FREQUENCIES AMONG PREVALENT ETHNIC GROUPS | | | | |
|---|---|---|---|---|
| HLA Allele | EUC | NAC | AFR | JPN |
| A2 | 45.3 | 46.6 | 27.3 | 43.2 |
| A29 | 7.4 | 8.1 | 12.3 | 0.4 |
| A31 | 5.4 | 6.2 | 4.4 | 15.3 |
| A32 | 8.8 | 7.1 | 3 | 0.1 |
| A33 | 3.3 | 3.4 | 9 | 13.1 |
| A28* | 7.7 | 9.9 | 16.6 | 1.1 |
| Abbreviations: EUC, European Caucasian; NAC, North American Caucasian; AFR, African blacks, JPN, Japanese. | | | | |

| | | | | |
|---|---|---|---|---|
| *A28 represents the two alleles Aw68 and Aw69 | | | | |

The peptides of the invention can be combined via linkage to form polymers (multimers), or can be formulated in a composition without linkage, as an admixture. Where the same peptide is linked to itself, thereby forming a homopolymer, a plurality of repeating epitopic units are presented. When the peptides differ, e.g., a cocktail representing different HBV subtypes, different epitopes within a subtype, different HLA restriction specificities, a peptide which contains T helper epitopes, heteropolymers with repeating units are provided. In addition to covalent linkages, noncovalent linkages capable of forming intermolecular and intrastructural bonds are included.

Linkages for homo- or hetero-polymers or for coupling to carriers can be provided in a variety of ways. For example, cysteine residues can be added at both the amino- and carboxy-termini, where the peptides are covalently bonded via controlled oxidation of the cysteine residues. Also useful are a large number of heterobifunctional agents which generate a disulfide link at one functional group end and a peptide link at the other, including N-succidimidyl-3-(2-pyridyldithio) proprionate (SPDP). This reagent creates a disulfide linkage between itself and a cysteine residue in one protein and an amide linkage through the amino on a lysine or other free amino group in the other. A variety of such disulfide/amide forming agents are known. See, for example, Immun. Rev. 62:185 (1982), which is incorporated herein by reference. Other bifunctional coupling agents form a thioether rather than a disulfide linkage. Many of these thioether forming agents are commercially available and include reactive esters of 6-maleimidocaproic acid, 2 bromoacetic acid, 2-iodoacetic acid, 4-(N-maleimido-methyl) cyclohexane-1-carboxylic acid and the like. The carboxyl groups can be activated by combining them with succinimide or 1-hydroxy-2-nitro-4-sulfonic acid, sodium salt. A particularly preferred coupling agent is succinimidyl 4-(N-maleimidomethyl) cyclohexane-1-carboxylate (SMCC). It will be understood that linkage should not substantially interfere with either of the linked groups to function as described, e.g., as an HBV cytotoxic T cell determinant, peptide analogs, or T helper determinant.

In another aspect the peptides of the invention can be combined or coupled with other peptides which present HBV T-helper cell epitopes, i.e., epitopes which stimulate T cells that cooperate in the induction of cytotoxic T cells to HBV. The T-helper cells can be either the T-helper 1 or T-helper 2 phenotype, for example. T-helper epitopes from HBV sequences have been identified at HBc1-20, having the sequence: Met-Asp-Ile-Asp-Pro-Tyr-Lys-Glu-Phe-Gly-Ala-Thr-Val-Glu-Leu-Leu-Ser-Phe-Leu-Pro [Seq ID No. 6]. Other T-helper epitopes are provided by peptides from the region HBc50-69, having the sequence Pro-His-His-Tyr-Ala-Leu-Arg-Gln-Ala-Ile-Leu-Cys-Trp-Gly-Glu-Leu-Met-Tyr-Leu-Ala [Seq ID No. 7], and from the region of HBc100-139, including HBc100-119 having the sequence Leu-Leu-Trp-Phe-His-Ile-Ser-Cys-Leu-Thr-Phe-Gly-Arg-Glu-Thr-Val-Ile-Glu-Tyr-Leu [Seq ID No. 8] (where Ile₁₁₆ is Leu in the HBV adw subtype), HBc117-131 having the sequence Glu-Tyr-Leu-Val-Ser-Phe-Gly-Val-Trp-Ile-Arg-Thr-Pro-Pro-Ala [Seq ID No. 9], and peptide HBc120-139 having the sequence Val-Ser-Phe-Gly-Val-Trp-Ile-Arg-Thr-Pro-Pro-Ala-Tyr-Arg-Pro-Pro-Asn-Ala-Pro-Ile [Seq ID No. 10]. See, Ferrari et al., J. Clin. Invest. 88:214-222 (1991), and U.S. Pat. 4,882,145.

The peptides of the invention can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co. (1984); Tam et al., J. Am. Chem. Soc. 105:6442 (1983); Merrifield, Science 232:341-347 (1986); and Barany and Merrifield, The Peptides, Gross and Meienhofer, eds., Academic Press, New York, pp. 1-284 (1979).

Alternatively, recombinant DNA technology may be employed wherein a nucleotide sequence which encodes a peptide of interest is inserted into an expression vector, transformed or transfected into an appropriate host cell and cultivated under conditions suitable for expression. These procedures are generally known in the art, as described generally in Sambrook et al., Molecular Cloning. A Laboratory Manual, Cold Spring Harbor Press, Cold Spring Harbor, New York (1982), and Ausubel et al., (ed.) Current Protocols in Molecular Biology, John Wiley and Sons, Inc., New York (1987), and U.S. Pat. Nos. 4,237,224, 4,273,875, 4,431,739, 4,363,877 and 4,428,941, for example. Thus, fusion proteins which comprise one or more peptide sequences of the invention can be used to present the HBV cytotoxic T cell determinants. For example, a recombinant polymerase protein of the invention is prepared in which the HBpol amino acid sequence is altered so as to more effectively present epitopes of peptide regions described herein to stimulate a cytotoxic T lymphocyte response. By this means a polypeptide is used which incorporates several T cell epitopes.

As the coding sequence for peptides of the length contemplated herein can be synthesized by chemical techniques, for example, the phosphotriester method of Matteucci et al., J. Am. Chem. Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Yeast or mammalian cell hosts may also be used, employing suitable vectors and control sequences.

The peptides of the present invention and pharmaceutical and vaccine compositions thereof are useful for administration to mammals, particularly humans, to treat and/or prevent HBV infection. As the peptides are used to stimulate cytotoxic T-lymphocyte responses to HBV infected cells, the compositions can be used to treat or prevent acute and/or chronic HBV infection.

For pharmaceutical compositions, the peptides of the invention as described above will be administered to an individual already infected with HBV. Those in the incubation phase or the acute phase of infection can be treated with the immunogenic peptides separately or in conjunction with other treatments, as appropriate. In therapeutic applications, compositions are administered to a patient in an amount sufficient to elicit an effective cytotoxic T lymphocyte response to HBV and to cure or at least partially arrest its symptoms and/or complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician, but generally range from about 1 µg to about 2,000 mg of peptide for a 70 kg patient, with dosages of from about 10 µg to about 100 mg of peptide being more commonly used, followed by booster dosages from about 1 µg to about 1 mg of peptide over weeks to months, depending on a patient's CTL response, as determined by measuring HBV-specific CTL activity in PBLs obtained from the patient. It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

Single or multiple administrations of the compositions can be carried out with dose levels and pattern being selected by the treating physician. In any event, the pharmaceutical formulations should provide a quantity of cytotoxic T-lymphocyte stimulatory peptides of the invention sufficient to effectively treat the patient.

For therapeutic use, administration should begin at the first sign of HBV infection or shortly after diagnosis in cases of acute infection, and continue until at least symptoms are substantially abated and for a period thereafter. In well established and chronic cases, loading doses followed by maintenance or booster doses may be required. The elicitation of an effective cytotoxic T lymphocyte response to HBV during treatment of acute hepatitis will minimize the possibility of subsequent development of chronic hepatitis, HBV carrier stage, and ensuing hepatocellular carcinoma.

Treatment of an infected individual with the compositions of the invention may hasten resolution of the infection in acutely infected individuals, about 90% of whom are capable of resolving the infection naturally. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic hepatitis and to stimulate the immune system of carriers to substantially reduce or even eliminate virus-infected cells. Those with chronic hepatitis can be identified as testing positive for virus from about 3-6 months after infection. As individuals may develop chronic HBV infection because of an inadequate (or absent) cytotoxic T lymphocyte response during the acute phase of their infection, it is important to provide an amount of immuno-potentiating peptide in a formulation and mode of administration sufficient to effectively stimulate a cytotoxic T cell response. Thus, for treatment of chronic hepatitis, a representative dose is in the range of about 1 µg to 1,000 mg, preferably about 5 µg to 100 mg for a 70 kg patient per dose. Administration should continue until at least clinical symptoms or laboratory indicators indicate that the HBV infection has been eliminated or substantially abated and for a period thereafter. Immunizing doses followed by maintenance or booster doses at established intervals, e.g., from one to four weeks, may be required, possibly for a prolonged period of time, as necessary to resolve the infection. For the treatment of chronic and carrier HBV infection it may also be desirable to combine the CTL peptides with other peptides or proteins that induce immune response to other HBV antigens.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the cytotoxic T-lymphocyte stimulatory peptides dissolved or suspended in an acceptable carrier, preferably an aqueous carrier. A variety of aqueous carriers may be used, e.g., water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

In some embodiments it may be desirable to include in the pharmaceutical composition at least one component which primes CTL. Lipids have been identified which are capable of priming CTL in vivo against viral antigens, e.g., tripalmitoyl-S-glycerylcysteinly-seryl-serine (P₃CSS), which can effectively prime virus specific cytotoxic T lymphocytes when covalently attached to an appropriate peptide. See, Deres et al., Nature 342:561-564 (1989). Peptides of the invention can be coupled to P₃CSS, for example, and the lipopeptide administered to an individual to specifically prime a cytotoxic T lymphocyte response to HBV. Further, as the induction of neutralizing antibodies can also be primed with P₃CSS conjugated to a peptide which displays an appropriate epitope, e.g., HBsAg epitopes, the two compositions can be combined to more effectively elicit both humoral and cell-mediated responses to HBV infection.

The concentration of cytotoxic T-lymphocyte stimulatory peptides of the invention in the pharmaceutical formulations can vary widely, i.e., from less than about 1%, usually at or at least about 10% to as much as 20 to 50% or more by weight, and will be selected primarily by fluid volumes, viscosities, etc., in accordance with the particular mode of administration selected.

Thus, a typical pharmaceutical composition for intravenous infusion could be made up to contain 250 ml of sterile Ringer's solution, and 100 mg of peptide. Actual methods for preparing parenterally administrable compounds will be known or apparent to those skilled in the art and are described in more detail in for example, Remington's Pharmaceutical Science, 17th ed., Mack Publishing Company, Easton, PA (1985).

The peptides of the invention may also be administered via liposomes, which serve to target the peptides to a particular tissue, such as lymphoid tissue or HBV-infected hepatic cells. Liposomes can also be used to increase the half-life of the peptide composition. Liposomes useful in the present invention include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor, prevalent among lymphoid cells, such as monoclonal antibodies which bind to the CD45 antigen, or with other therapeutic or immunogenic compositions. Thus, liposomes filled with a desired peptide of the invention can be directed to the site of lymphoid or hepatic cells, where the liposomes then deliver the selected therapeutic/immunogenic peptide compositions, Liposomes for use in the invention are formed from standard vesicle-forming lipids, which generally include neutral and negatively charged phospholipids and a sterol, such as cholesterol. The selection of lipids is generally guided by consideration of, e.g., liposome size and stability of the liposomes in the blood stream. A variety of methods are available for preparing liposomes, as described in, e.g., Szoka et al., Ann. Rev. Biophys. Bioeng. 9:467 (1980), U.S. Patent Nos. 4,235,871, 4,501,728, 4,837,028, and 5,019,369. For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, the mode of administration, the peptide being delivered, the stage of disease being treated, etc.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the cytotoxic T-lymphocyte stimulatory peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included as desired, e.g., lecithin for intranasal delivery.

In another aspect the present invention is directed to vaccines which contain as an active ingredient an immunogenically effective amount of a cytotoxic T-lymphocyte stimulating peptide as described herein. The peptide(s) may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or cytotoxic T cells that react with different antigenic determinants of HBV. Useful carriers are well known in the art, and include, e.g., keyhole limpet hemocyanin, thyroglobulin, albumins such as human serum albumin, tetanus toxoid, polyamino acids such as poly(D-lysine:D-glutamic acid), and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, cytotoxic T lymphocyte responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of cytotoxic T-lymphocytes specific for HBV antigen, and the host becomes at least partially immune to HBV infection, or resistant to developing chronic HBV infection.

Vaccine compositions containing the peptides of the invention are administered to a patient susceptible to or otherwise at risk of HBV infection to enhance the patient's own immune response capabilities. Such an amount is defined to be a "immunogenically effective dose." In this use, the precise amounts again depend on the patient's state of health and weight, the mode of administration, the nature of the formulation, etc., but generally range from about 1.0 µg to about 500 mg per 70 kilogram patient, more commonly from about 50 µg to about 200 mg per 70 kg of body weight. The peptides are administered to individuals of an appropriate HLA type, e.g., for vaccine compositions of peptides from the region of HBpol61-69 [Seq ID No. 1], Gly-Leu-Tyr-Ser-Ser-Thr-Val-Pro-Val; HBpol 455-463 [Seq ID No. 2], Gly-Leu-Ser-Arg-Tyr-Val-Ala-Arg-Leu; HBpol551-559 and HBpol655-663; HBpol 773-782 [Seq ID No. 3], Ile-Leu-Arg-Gly-Thr-Ser-Phe-Val-Tyr-Val; HBpol803-811 [Seq ID No. 4], Ser-Leu-Tyr-Ala-Asp-Ser-Pro-Ser-Val; or HBpol816-824 [Seq ID No. 5], Phe-Leu-Leu-Ser-Leu-Gly-Ile-His-Leu, these will be administered to at least HLA-A2 individuals. For peptides from HBpol151-160, these will be administered to at least HLA-A1 individuals. Vaccines comprising peptides from HBpol575-583 will be administered to at least HLA-A2.1 individuals. Vaccines comprising peptides from HBpol575-583 will be administered to at least HLA-A2.1 individuals. Vaccines comprising peptides from HBpol108-116, HBpol139-147, HBpol152-161, and HBpol748-757 will be administered to at least HLA-A3 individuals, and/or A24 individuals in the case of HBpol748-757. The peptides HBpol4-13, HBpol505-514, and HBpol758-766 will be administered to at least HLA-A24 individuals.

In some instances it may be desirable to combine the peptide vaccines of the invention with vaccines which induce neutralizing antibody responses to HBV, particularly to HBV envelope and/or core antigens, such as recombinant HBV env- and/or nucleocapside-encoded antigens or vaccines prepared from purified plasma preparations obtained from HBV-infected individuals. A variety of HBV vaccine preparations have been described, and are based primarily on HBsAg and polypeptide fragments thereof. For examples of vaccines which can be formulated with the peptides of the present invention, see generally, EP 154,902 and EP 291,586, and U.S. Pat. Nos. 4,565,697, 4,624,918, 4,599,230, 4,599,231, 4,803,164, 4,882,145, 4,977,092, 5,017,558 and 5,019,386. The vaccines can be combined and administered concurrently, or as separate preparations.

For therapeutic or immunization purposes, the peptides of the invention can also be expressed by attenuated viral hosts, such as vaccinia. This approach involves the use of vaccinia virus as a vector to express nucleotide sequences that encode the HBV peptides of the invention. Upon introduction into an acutely or chronically HBV-infected host or into a non-infected host, the recombinant vaccinia virus expresses the HBV peptide and thereby elicits a host cytotoxic T lymphocyte response to HBV. Vaccinia vectors and methods useful in immunization protocols are described in, e.g., U.S. Patent No. 4,722,848, incorporated herein by reference. Another vector is BCG (bacille Calmette Guerin). BCG vectors are described in Stover et al. (Nature 351:456-460 (1991)). A wide variety of other vectors useful for therapeutic administration or immunization of the peptides of the invention, e.g., Salmonella typhi vectors and the like, will be apparent to those skilled in the art from the description herein.

The compositions and methods of the claimed invention may be employed for ex vivo therapy. By ex vivo therapy is meant that therapeutic or immunogenic manipulations are performed outside the body. For example, lymphocytes or other target cells may be removed from a patient and treated with high doses of the subject peptides, providing a stimulatory concentration of peptide in the cell medium far in excess of levels which could be accomplished or tolerated by the patient. Following treatment to stimulate the CTLs, the cells are returned to the host to treat the HBV infection. The host's cells may also be exposed to vectors which carry genes encoding the peptides, as described above. Once transfected with the vectors, the cells may be propagated in vitro or returned to the patient. The cells which are propagated in vitro may be returned to the patient after reaching a predetermined cell density.

In one method, ex vivo CTL responses to a HBV are induced by incubating in tissue culture a patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an HBV infected cell). To optimize the in vitro conditions for the generation of specific cytotoxic T cells, the culture of stimulator cells is typically maintained in an appropriate serum-free medium. Peripheral blood lymphocytes are conveniently isolated following simple venipuncture or leukapheresis of normal donors or patients and used as the responder cell sources of CTLp. In one embodiment, the appropriate APC are incubated with about 10-100 µM of peptide in serum-free media for 4 hours under appropriate culture conditions. The peptide-loaded APC are then incubated with the responder cell populations in vitro for 5 to 10 days under optimized culture conditions.

Positive CTL activation can be determined by assaying the cultures for the presence of CTLs that kill radiolabeled target cells, both specific peptide-pulsed targets as well as target cells expressing endogenously processed form of the HBV polymerase antigen from which the peptide sequence was derived. Specificity and MHC restriction of the CTL of a patient can be determined by a number of methods known in the art. For instance, CTL restriction can be determined by testing against different peptide target cells expressing appropriate or inappropriate human MHC class I. The peptides that test positive in the MHC binding assays and give rise to specific CTL responses are identified as immunogenic peptides.

The induction of CTL in vitro requires the specific recognition of peptides that are bound to allele specific MHC class I molecules on APC. Peptide loading of empty major histocompatibility complex molecules on cells allows the induction of primary CTL responses. Since mutant cell lines do not exist for every human MHC allele, it may be advantageous to use a technique to remove endogenous MHC-associated peptides from the surface of APC, followed by loading the resulting empty MHC molecules with the immunogenic peptides of interest. The use of non-transformed, non-infected cells, and preferably, autologous cells of patients as APC is desirable for the design of CTL induction protocols directed towards development of ex vivo CTL therapies. Typically, prior to incubation of the APCs with the CTLp to be activated, an amount of antigenic peptide is added to the APC or stimulator cell culture, of sufficient quantity to become loaded onto the human Class I molecules to be expressed on the surface of the APCs. Resting or precursor CTLs are then incubated in culture with the appropriate APCs for a time period sufficient to activate the CTLs. Preferably, the CTLs are activated in an antigen-specific manner. The ratio of resting or precursor CTLs to APCs may vary from individual to individual and may further depend upon variables such as the amenability of an individual's lymphocytes to culturing conditions and the nature and severity of the disease condition or other condition for which the described treatment modality is used. Preferably, however, the CTL:APC ratio is in the range of about 30:1 to 300:1. The CTL/APC may be maintained for as long a time as is necessary to stimulate a therapeutically useable or effective number of CTL.

Activated CTL may be effectively separated from the APC using one of a variety of known methods. For example, monoclonal antibodies specific for the APCs, for the peptides loaded onto the stimulator cells, or for the CTL (or a segment thereof) may be utilized to bind their appropriate complementary ligand. Antibody-tagged molecules may then be extracted from the admixture via appropriate means, e.g., via well-known immunoprecipitation or immunoassay methods.

Effective, cytotoxic amounts of the activated CTLs can vary between in vitro and in vivo uses, as well as with the amount and type of cells that are the ultimate target of these killer cells. The amount will also vary depending on the condition of the patient and should be determined via consideration of all appropriate factors by the practitioner. Preferably, however, about 1 X 10⁶ to about 1 X 10¹², more preferably about 1 X 10⁸ to about 1 X 10¹¹, and even more preferably, about 1 X 10⁹ to about 1 X 10¹⁰ activated CD8+ cells are utilized for adult humans, compared to about 5 X 10⁶ - 5 X 10⁷ cells used in mice.

Methods of reintroducing cellular components are known in the art and include procedures such as those exemplified in U.S. Patent No. 4,844,893 to Honsik, et al. and U.S. Patent No. 4,690,915 to Rosenberg. For example, administration of activated CTLs via intravenous infusion is typically appropriate.

The peptides may also find use as diagnostic reagents. For example, a peptide of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic HBV infection.

The following examples are offered by way of illustration, not by way of limitation.

### EXAMPLE I

### HLA-Restricted CTL Response TO HBV Polymerase Epitopes

This Example describes the identification of an HLA-A2 restricted CTL response to two HBV polymerase peptides in a patient with acute viral hepatitis. The epitopes are present in amino acid sequences HBpol₆₁₋₆₉ [Seq ID No. 1] Gly-Leu-Tyr-Ser-Ser-Thr-Val-Pro-Val (GLYSSTVPV) (also designated peptide 927.32) and HBpol₈₀₃₋₈₁₁ (Seq ID No. 4] Ser-Leu-Tyr-Ala-Asp-Ser-Pro-Ser-Val (SLYADSPSV) (also designated peptide 927.27).

The CTL induced by the HBpol peptides were identified in PBMCs from a patient with acute hepatitis according to the procedure set forth in Example VI of pending application WO94/19011 except that the PMBCs were stimulated with individual peptides rather than peptide mixtures. The resulting CTL lines and/or clones were then tested for the ability to kill HLA-A2 matched target cells that were either pulsed with the peptide or that expressed the corresponding endogenous polymerase antigen (Vpol or EBO-pol). Construction of the vaccinia based Vpol and Epstein-Barr virus based EBO-pol constructs was as described in Example II of USSN WO94/19011.

As shown in Fig. 1, both peptides HBpol₈₀₃₋₈₁₁ and HBpol₆₁₋₆₉ stimulated CTL responses in a patient (HLA-A2⁺) using target cells pulsed with peptide, whereas other peptides 927.24 (WILRGTSFR) [Seg ID No. 23] and 927.30 (DLNLGNLMV) [Seq ID No. 24] and media controls did not stimulate the specific CTL response. The ability of the HBpol₈₀₃₋₈₁₁ specific clones to recognize endogenously synthesized polymerase antigen (Vpol and EBO-pol) is shown in Fig. 2. Two clones, designated Be.27-1A1 and Be.27-1A5, were identified that recognized the HBpol₈₀₃₋₈₁₁ peptide. As shown in Fig. 3, CTL responses to HBpol₆₁₋₆₉ and HBpol₈₀₃₋₈₁₁ were shown with target cells pulsed with homologous peptide, but only the HBpol₈₀₃₋₈₁₁ clone showed a response to endogenously synthesized Vpol antigen.

### EXAMPLE II

This example demonstrates that acutely infected patients with clinically apparent viral hepatitis develop an HLA class I restricted CTL response to multiple epitopes in the conserved functional domains of the HBV polymerase protein, while persistently infected patients with chronic hepatitis and normal uninfected controls do not.

Nine HLA-A2 positive patients with acute hepatitis B, nine patients with chronic hepatitis B and ten healthy uninfected subjects were studied (Table II). The diagnosis of acute hepatitis B was based on clinical and biochemical evidence of acute liver injury according to standard diagnostic criteria, together with serological evidence of acute HBV infection, i.e., hepatitis B surface antigen (HBsAg, hepatitis B e antigen (HBeAg) and IgM anti-HBc antibody (IgM HBc-Ab), and the absence of serologic evidence of hepatitis delta or hepatitis C virus infection. Six of the nine patients recovered completely with normalization of serum transaminases and clearance of HBsAg and HBeAg within four months of initial diagnosis; the remaining three patients were lost to follow up. All patients with chronic hepatitis B were repeatedly serologic positive for HBsAg for more than six months and displayed mildly to moderately elevated serum ALT activity. Normal controls had no clinical history of HBV infection and were serologically negative for all HBV markers.

The amino acid sequence of HBV polymerase was screened for 9-mers and 10-mers containing the HLA-A2 allele specific binding motif. This search yielded 220 candidate peptides. Out of this group 44 peptides were selected based on conservation in at least 4 of the 7 HBV adw sequences in the GenBank Database. Lyophilized peptides were reconstituted at 20 mg/ml in DMSO and diluted to 1 mg/ml with RPMI 1640 medium.

**Table II.**

| Characteristics of Subjects Studied | | | |
|---|---|---|---|
| Subject | Sex | Diagnosis | HLA class I haplotype |
| A-1 | Male | Acute | A2, A24, B51, B53, Cw1 |
| A-2 | Male | Acute | A2, A63, B44, B54, Cw7 |
| A-3 | Male | Acute | A2, A24, B27, B71/72, Cw1, Cw4 |
| A-4 | Female | Acute | A2, A31, B51, B6, Cw3 |
| A-5 | Male | Acute | A2, A30, B44, B35, CW4, Cw7 |
| A-6 | Female | Acute | A2, A69, B53, Cw4 |
| A-7 | Female | Acute | A2, A74, B62, B57, Cw3, Cw6 |
| A-8 | Male | Acute | A2, A68, B58, B27, Cw1, Cw6 |
| A-9 | Male | Acute | A2, A30, B35, Cw5 |
| | | | |
| CH-1 | Male | Chronic | A2, A23, B44 |
| CH-2 | Male | Chronic | A2, A1, B8, B44, Cw7, Cw4 |
| CH-3 | Male | Chronic | A2, A68, B59, B44, Cw5, Cw7 |
| CH-4 | Male | Chronic | A2, B7801, B13, Cw7 |
| CH-5 | Male | Chronic | A2, A30, B44, B13, CW6 |
| CH-6 | Male | Chronic | A2, A34, B8, B27, Cw7 |
| CH-7 | Male | Chronic | A2, A33, B62, B67, Cw8 |
| CH-8 | Male | Chronic | A2, A69, B41, B52 |
| CH-9 | Male | Chronic | A2, A25, B18, Cw6 |
| | | | |
| N-1 | Female | Normal | A2, A32, B18, B60, Cw3, Cw7 |
| N-2 | Male | Normal | A2, B44, Cw7 |
| N-3 | Male | Normal | A2, A1, B8, B18, Cw7 |
| N-4 | Female | Normal | A2, B44, Cw63 |
| N-5 | Male | Normal | A2, A23, B5, B58, Cw2, Cw6 |
| N-6 | Male | Normal | A2, B35, B56, Cw1, cw3 |
| N-7 | Male | Normal | A2, A11, B8, B62, Cw4, Cw7 |
| N-8 | Female | Normal | A2, A3, B7, B60, Cw3, Cw7 |
| N-9 | Male | Normal | A2, A11, B35, B44, Cw4 |
| N-10 | Male | Normal | A2, A3, B13, B35, Cw4 |

The binding affinity of the peptides to the class I molecule was determined by competitive binding assays using the radiolabeled peptide FLPSDYFPSV [Seq ID No. 25] representing HBc18-27. The peptide was iodinated to a specific activity of 5-10 x 10⁷ cpm/mol by the chloramine T method of Buus et al., Science 235: 1353 (1987). Purified class I molecules (10 to 50 nM) were incubated at room temp. with various doses of the peptides, together with 5 to 10 nM of the labeled peptide and 1 µM human β2-microglobulin in PBS, pH 7.0, 0.05% NP-40, 1 mM PMSF, 1.3 mM 1,10-phenanthroline, 73 µM pepstatin A, 8 mM EDTA, and 200 µM TLCK. After 48 hrs., class I-peptide complexes were separated from free peptide by gel filtration on either a TSK2000 (7.8mm x 15cm) column eluted with PBS pH 6.5, 0.5% NP-40, 0.1% NaN₃, or a Sephadex G-50 column (22ml bed volume) eluted with the same buffer at pH 7.0. Class I-bound and free radioactivity was measured and the doses of peptides yielding 50% inhibition of the binding of the labeled peptide (IC50) were calculated. Before conducting inhibition assays, purified class I molecules were titered in the presence of a fixed amount of labeled peptide to determine the concentration necessary to bind 10 to 30% of the total radioactivity added. All subsequent inhibition assays were then performed using these class I concentrations. Each peptide was tested in two to four independent experiments.

Fifteen of the peptides displayed an HIA-A2.1 binding affinity ratio greater than 0.01. (Table III), a threshold below which most peptides are not immunogenic. In addition two peptides which contain HLA-A2 restricted CTL epitopes were included for comparison, HBc18-27 and HBs335-343.

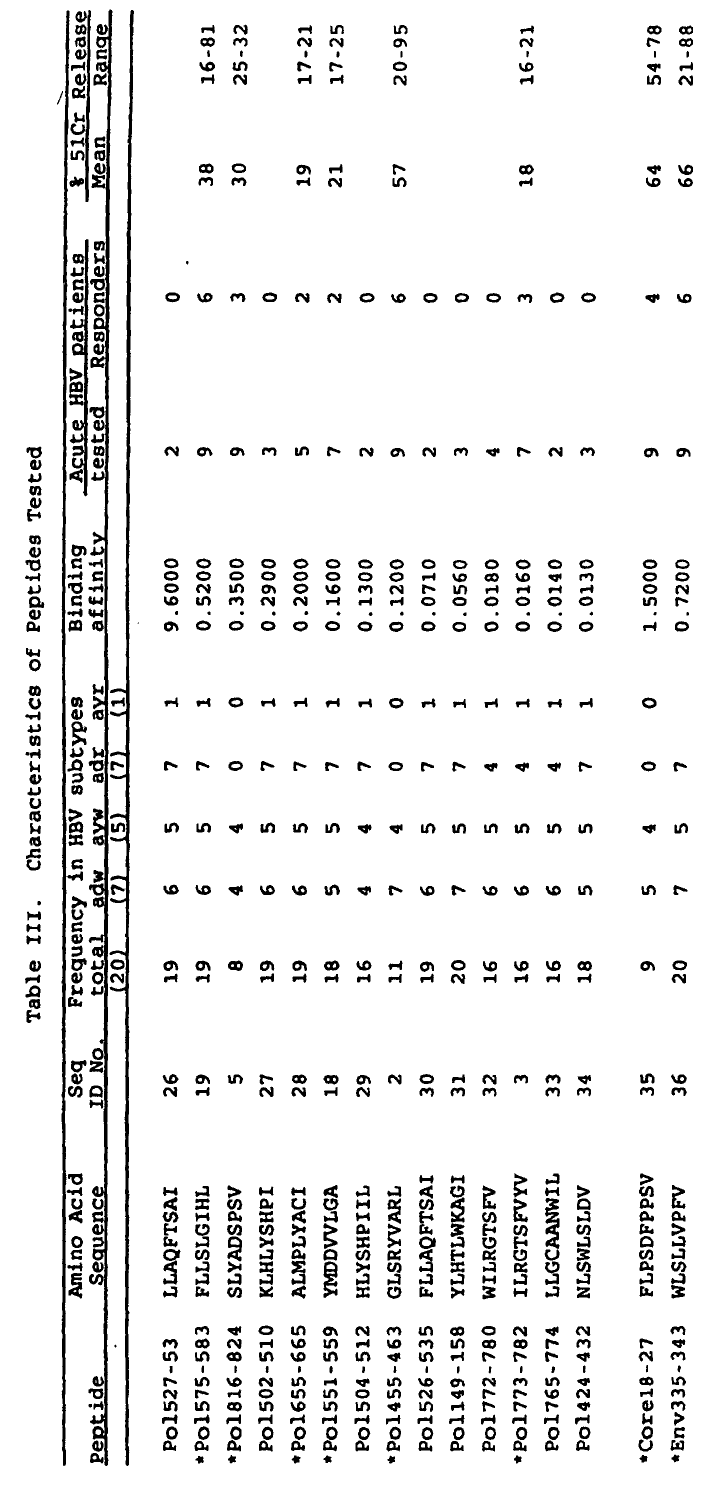

To stimulate PBMC with the selected synthetic peptides and rHBcAg, PBMC from patients and normal donors were separated on Ficoll-Histopaque density gradients, washed three times in Hanks Balanced Salt Solution (HBSS), resuspended in RPMI 1640 supplemented with L-glutamine (2 mM), gentamicin (10 µg/ml), and 10% heat-inactivated human AB serum and plated in a 24-well plate at 4 x 10⁶ cells/well. rHBcAG (Biogen, Cambridge, MA) was added to the cell cultures at 1 µg/ml and the synthetic peptides at 10 µg/ml. In some of the studies with healthy uninfected blood donors rHBcAg was either omitted or replaced by 10 µg/ml tetanus toxoid (Connaught Laboratories, Swiftwater, PA) since these individuals had not been previously exposed to HBV and did not benefit from rHBcAg-induced T cell help. On days 3 and 10, 1 ml of RPMI with 10% human AB serum and rIL-2 at 10 U/ml final concentration was added to each well. On day 7, the cultures were restimulated with peptide, rIL-2 and irradiated (3000 rad) autologous feeder cells and they were tested for cytotoxic activity on day 14. Selected cultures that displayed peptide specific cytolytic activity were separated into CD4+ and CD8+ populations by panning onto anti-CD4 coated flasks (Applied Immunosciences, Santa Clara, CA) and restimulated as described above.

CTL lines were established as described above and enriched in highly cytotoxic CD8+ CTLs by cloning at 10 and 3 cells per well in 96-well microwell plates in the presence of 0.5 µg/ml CD3-specific monoclonal antibody (Coulter Immunology, Hialeah, FL), rIL-2 (100 U/ml) and 10⁵ irradiated (3000 rad) allogeneic PBMC. HBV specific clones were established by cloning at 1 and 0.3 cells per well in the same way. Growing cultures were tested for cytotoxic activity against peptide-primed target cells on day 17 and cytotoxic lines and clones were expanded in a 24-well plate and restimulated every 7 to 10 days as described above.

For the cytotoxicity assays, target cells consisted of either 1) allogeneic HLA-matched and mismatched B-LCL (Amer. Soc. Histocompat. Immunogenetics, Boston, MA), incubated overnight with synthetic peptides at 10 µg/ml; 2) stable B-LCL transfectants that express HBsAg or HBpolAg produced by transfection of the EBV-transformed B-LCL with a panel of EBV-based expression vectors that contain the corresponding coding regions of the ayw subtype (Guilhot et al., J.Virol. 66: 2670 (1992); or 3) B-LCL infected with recombinant vaccinia viruses (a recombinant vaccinia virus construct that encodes the HBV polymerase protein (Vpol) was produced by insertion of a 2766 fragment representing nucleotides 2290-1874 of the HBV genome (ayw subtype) into the Sma I site of the pSCII vector by standard techniques as described in Chakrabarti et al., Mol. Cell. Biol. 5: 3403 (1985). Vaccinia-infected targets were prepared by infection of 10⁶ cells at 50 PFU/cell on a rocking plate at room temp. for 1 h followed by a single wash and overnight incubation at 37°C. Target cells were then labeled with 100 µCi of ⁵¹Cr (Amersham Corp., Arlington Heights , IL) for 1 h and washed four times with HBSS. Cytolytic activity was determined in a standard 4-h ⁵¹Cr release assay using U-bottomed 96-well plates containing 5,000 targets/well. Stimulated PBMC from patients and normal controls were performed in duplicate. Percent cytotoxicity was determined from the formula 100 x ((experimental release - spontaneous release) / (maximum release - spontaneous release)). Maximum release was determined by lysis of targets by detergent (1% Triton X-100; Sigma Chemical Co., St. Louis, MO). Spontaneous release was <20% of maximal release in all assays. The assay was considered positive if the specific ⁵¹Cr release from target cells containing antigen was ≥ 15% higher than the nonspecific ⁵¹Cr release from antigen nonspecific ⁵¹Cr release from antigen negative target cells and the nonspecific lysis was less than 15% of maximum.

As shown in Table III, eight of the nine acutely infected patients responded to at least one of the polymerase peptides and, as can be seen from Table III, six of the peptides were recognized by at least one patient, suggesting that they represented HLA-A2 restricted epitopes. The HLA binding ratio of 5 of 6 of these peptides was greater than 0.1, supporting a direct relationship between binding affinity and immunogenicity even among this group of high affinity peptides.

The HLA-A2 binding affinity of a peptide did not appear to be the only requirement for immunogenicity since the peptide (LLAQFTSAI) [Seq ID No. 26] with the highest binding affinity (9.600) did not elicit an immune response while one with a 600-times lower affinity (0.016) did. To exclude the possibility that this extremely high affinity peptide may have triggered potentially responsive CTL precursors to undergo apoptosis, PBMC were also stimulated with lower concentrations of this peptide (0.3, 1, 3 and 10 µg/ml) without inducing a CTL response, suggesting that nonresponsiveness to this and other high affinity peptides is probably due to other mechanisms.

The CTL responses of nine acutely infected patients who responded to one or more polymerase peptides are summarized in Fig. 5. Five of these patients also recognized the two control peptides, HBC18-27 and HBenv335-343, while one patient recognized only HBenv335-343, and one patient responded to neither. These results demonstrate the clonality and multispecificity of the CTL response against the polymerase protein during acute viral hepatitis. Importantly, nine of the 10 uninfected controls responded to any of the peptides used in this example (nine of these controls are shown in Fig. 5), suggesting that the CTL responses observed in the acutely infected patients represented in vitro secondary responses that were primed by exposure to infected cells in vivo. None of the nine patients with chronic hepatitis produced a response, suggesting that the vigor of the HBV specific CTL response has a role in determining which patients will clear the virus and which patients will not.

Having identified two HLA-A2 patients (A-1 and A-2) with acute hepatitis who responded strongly to HBpol575-583 and HBpol455-463 and HBpol816-824 (Table III), these patients and peptides were chosen for further analysis. After two weeks of in vitro stimulation, selected cultures that displayed peptide specific CTL responses were enriched for CD4+ and CD8+ subsets by panning using positive and negative selection, respectively, and they were restimulated with peptide and tested for recognition of endogenously processed polymerase antigen after one additional week of culture. As shown in Fig. 6, the CTL response to these epitopes was mediated by CD8+ T cells since only the CD8+ fraction of each cell line recognized target cells that were either pulsed with the corresponding peptide or stably transfected with the polymerase expression vector. These results suggest that the peptides represent the native epitopes that are produced by the cellular processing of the polymerase protein, and that they are presented in the context of class I HLA molecules.

To obtain pure CD8+ cell lines and to characterize the T cell response at the clonal level, each of the three responding cell lines was cloned by limiting dilution in the presence of anti-CD3, irradiated allogeneic PBL and IL-2. All of the derivative cytotoxic lines were highly enriched in CD8+ cells as determined by FACS analysis (0.5 - 1.0 x 10⁶ cells were washed once in PBS with 5% BSA and 0.02% sodium azide, the pelleted cells were then stained with a fluorescent probe-conjugated anti-CD4 and anti-CD8 monoclonal antibody (Leu3a or Leu2a), and similarly labeled control antibody for 30 min. at 4°C, and after 3 washes in PBS with 5% BSA and 0.02% sodium azide, cells were analyzed with a FACScan flow cytometer). Furthermore, 5 of the 6 HBpol455-463 specific CTL clones derived in this manner also consisted of CD8+ cells.

Four highly cytotoxic long term CTL lines and two clones specific for HBpol455-463 pulsed targets were chosen for further analysis (Fig. 7). The strength of the cytotoxic activity was assessed by varying the amount of the peptide used to pulse the target cells and by varying the effector to target ratios. The CTL displayed peptide dose dependent cytotoxic activity that recognized targets pulsed with peptide concentrations as low as 10 nM (Table IV), and they efficiently lysed both peptide pulsed and vaccinia-pol infected targets at E:T ratios as low as 1.6:1 (Fig. 7). Target cells pulsed with no peptide or with an irrelevant peptide (Table V), which is an HLA-A2 restricted epitope in HCV-infected patients, were not lysed, now were cells infected by the control recombinant vaccinia virus that expresses the HBV envelope protein, indicating the specificity of the CTL.

**Table IV.**

| Recognition GLSRYVARL-pulsed JY-EBV by CTL is peptide-dose dependent [Seq ID No. 2] | | | | | | |
|---|---|---|---|---|---|---|
| Patient | Line | JY-EBV pulsed with | | | | |
| | | 10 µM GLSRYVARL | 1 µM GLSRYVARL | 0.1 µM GLSRYVARL | 0.01 µM GLSRYVARL | no peptide |
| A-1 | 67-68 | 41% | 26% | 19% | 13% | 5% |
| A-2 | 10 | 75% | 56% | 52% | 25% | 12% |
| A-2 | 30 | 69% | 40% | 40% | 19% | 6% |

**Table V.**

| Induction of CTL with variant peptides to GLSRYVARL (Seq ID No. 2] and an HCV epitope KLVALGINAV [Seq ID No. 37] | | | | | |
|---|---|---|---|---|---|
| Peptide During CTL-Induction | % Specific Cytotoxicity Against JY-EBV Preincubated with | | | | |
| | GLSRYVARL | GLPRYVARL | SGLSRYVARL | GLSRYVARLS | KLVALGINAV |
| | Seq ID 2 | Seq ID 38 | Seq ID 39 | Seq ID 40 | Seq ID 37 |
| GLSRYVARL | 54% | 18% | 40% | 41% | 2% |
| GLPRYVARL | 1% | 0% | 1% | 0% | |
| SGLSRYVARL | 0% | 0% | 0% | 0% | |
| GLSRYVARLS | 1% | 1% | 0% | 0% | |

To identify the restriction element used by the HBpol455-463 specific CTL, cytotoxic lines and clones from patients A-1 and A-2 were tested against allogeneic EBV-B cell lines sharing individual HLA class I alleles with the effector cells. As shown in Fig. 8, not only was HLA-A2 the sole class I allele shared by these two patients, but their CTL only lyse peptide pulsed target cells that share this allele. Thus, HBpol455-463 specific CTLs from both patients are HLA-A2 restricted.

Peptides containing carboxy- and amino-terminal truncations and elongations of the HBpol455-463 sequence were synthesized to determine the optimal length and the precise termini of the epitope. As shown in Fig. 9A, truncation of Gly455 or Leu463 greatly reduced the HLA binding affinity of the peptides and totally abrogated their recognition by CTL induced by the original peptide HBpol455-463. Elongation of this peptide by adding a single Ser residue normally present upstream of the amino terminus or downstream of the carboxy-terminus of HBpol455-463 did not diminish its recognition by CTL (Fig. 9A), and may have even increased recognition, despite the fact that the HLA-A2 binding affinity of the extended peptides was reduced 4-10 fold relative to the original peptide (Fig. 9A). The Ser-extended peptides did not induce CTL, as shown in Table V.

Direct sequencing of the PCR products amplified from the serum of 5 of the 9 patients with acute hepatitis B by nested PCR demonstrated that the deduced HBV amino acid sequence was identical to GLSRYVARL [Seq ID No. 2] in these patients. The sequence is present in 7/7 and 4/5 adw and ayw subtype sequences in GenBank. The amino acid sequence of the remaining ayw isolate in the database is GVSRYVARL [Seq ID No. 41], while the sequence of 6/7 adr and 1/1 ayr isolates is GLPRYVARL [Seq ID No. 42] and the sequence of the remaining adr isolate is GLPRYVVCL [Seq ID No. 43].

Peptides containing sequences of these different viral subtypes were tested for recognition by GLSRYVARL-stimulated [Seq ID No. 2] PBMC to assess cross-reactivity of the CTL response. None of the variants was efficiently recognized by the CTL. GLSRYVVCL [Seq ID No. 44] was not recognized, even at very high peptide concentration, despite the fact that its HLA-A2.1 binding affinity was greater than the prototype peptide GLSRYVARL [Seq ID No. 2]. Thus, Ser457, Ala461 and Arg462 may represent T cell receptor contact sites (epitope residues) in this peptide. A substitution in Ser457 in GLPRYVARL [Seq ID No. 42] variant yielded more than a 10-fold reduction in its recognition by the CTL, while decreasing the HLA binding affinity 2-fold.

The GVSRYVARL [Seq ID No. 41] variant which contained the substitution at Leu456, a presumptive HLA contact site (agretope residue), was poorly recognized by the CTL, commensurate with the 9-fold reduction in its HLA-A2 binding affinity. However, the amino- and carboxy-terminally extended peptides described above were well recognized by the CTL despite the fact that they displayed comparably reduced HLA-A2 binding affinities (Fig. 9A). This suggests that Leu456 not only serves as an agretope residue, but may also influence the T cell receptor binding affinity of the peptide.

From the foregoing it will be appreciated that, although specific embodiments of the invention have been described herein for purposes of illustration, various modifications may be made. Accordingly, the invention is not limited except as by the appended claims.

## Claims

1. A CTL-inducing peptide comprising from eight to thirteen amino acids, wherein the CTL-inducing peptide comprises

2. The CTL-inducing peptide of claim 1, which comprises from nine to eleven amino acids.

3. The peptide of claim 1 or claim 2, suspended in pharmaceutically acceptable carrier which comprises a liposome.

4. The CTL-inducing peptide of any one of the preceding claims, wherein the peptide also contains a T helper epitope.

5. The peptide of any one of the preceding claims, conjugated to a immunogenic lipid carrier.

6. A method for stimulating a cytotoxic T lymphocyte response to hepatitis B virus which comprises exposing cytotoxic T lymphocytes of a host to a peptide as claimed in any one of the preceding claims.

7. The method of claim 6, wherein the host cells exposed to the CTL-inducing peptides are HLA-A2 restricted.

8. The method of claim 7, wherein the cytotoxic T cells are removed from the host prior to being exposed to the CTL-inducing peptide.

9. A method for stimulating a cytotoxic T lymphocyte response to hepatitis B virus which comprises exposing cytotoxic T lymphocytes of a host to a peptide which contains a CTL epitope and which comprises from eight to thirteen amino acids, wherein the CTL-inducing peptide is selected from a peptide as claimed in any one of claims 1 to 5, or and wherein the host cells exposed to the CTL-inducing peptides are HLA-A2 restricted, and wherein the cytotoxic T cells are removed from the host prior to being exposed to the CTL-inducing peptide.

10. Use of a CTL-inducing peptide which is either:
(a) a peptide as claimed in any one of claims 1 to 5, or
(b) a peptide which contains a CTL epitope and which comprises from eight to thirteen amino acids, selected from:
for the preparation of a medicament for use in a method for stimulating a cytotoxic T lymphocyte response to hepatitis B virus, which method comprises:
(i) exposing cytotoxic T lymphocytes of a host to the peptide, wherein the host cells exposed to the CTL-inducing peptides are HLA-A2 restricted, and wherein the cytotoxic T cells are removed from the host prior to being exposed to the CTL-inducing peptide, and
(ii) returning the stimulated CTLs to the host.

11. The method of any one of claims 6 to 9, or the use of claim 10, wherein the host has acute or chronic hepatitis B infection or is a hepatitis B carrier.

12. The method of any one of claims 6 to 9, or the use of claim 10, wherein the CTL-inducing peptide is administered prophylactically to the host's cells.

13. The method of any one of claims 6 to 9 or claim 12, or the use of claim 10, wherein the CTL-inducing peptide is administered to the host with a second peptide which elicits a T helper response to HBV.

14. The method or use of claim 13 , wherein the CTL-inducing peptide and the T helper inducing peptide are linked.

## Patentansprüche

1. CTL-induzierendes Peptid, das 8 bis 13 Aminosäuren umfasst, worin das CTL-induzierende Peptid umfasst:

2. CTL-induzierendes Peptid nach Anspruch 1, das 9 bis 11 Aminosäuren umfasst.

3. Peptid nach Anspruch 1 oder 2, das in einem pharmazeutisch annehmbarem Träger suspendiert ist, der ein Liposom umfasst.

4. CTL-induzierendes Peptid nach einem der vorangegangenen Ansprüche, worin das Peptid auch ein T-Helfer-Epitop enthält.

5. Peptid nach einem der vorangegangenen Ansprüche, das an einen immunogenen Lipidträger konjugiert ist.

6. Verfahren zum Stimulieren einer zytotoxischen T-Lymphozyten-Reaktion auf Hepatitis B-Virus, welches das Aussetzen von zytotoxischen T-Lymphozyten eines Wirts gegenüber einem Peptid nach einem der vorangegangenen Ansprüche umfasst.

7. Verfahren nach Anspruch 6, worin die Wirtszellen, die den CTL-induzierenden Peptiden ausgesetzt werden, HLA-A2-restringiert sind.

8. Verfahren nach Anspruch 7, worin die zytotoxischen T-Zellen aus dem Wirt entfernt werden, bevor sie dem CTL-induzierenden Peptid ausgesetzt werden.

9. Verfahren zum Stimulieren einer zytotoxischen T-Lymphozyten-Reaktion auf Hepatitis B-Virus, welches das Aussetzen von zytotoxischen T-Lymphozyten eines Wirts gegenüber einem Peptid umfasst, das ein CTL-Epitop enthält und das 8 bis 13 Aminosäuren umfasst, worin das CTL-induzierende Peptid aus einem Peptid nach einem der Ansprüche 1 bis 5 oder ausgewählt ist, worin die den CTL-induzierenden Peptiden ausgesetzten Wirtszellen HLA-A2-restringiert sind und worin die zytotoxischen T-Zellen aus dem Wirt entfernt werden, bevor sie dem CTL-induzierenden Peptid ausgesetzt werden.

10. Verwendung eines CTL-induzierenden Peptids, das entweder:
(a) ein Peptid nach einem der Ansprüche 1 bis 5 oder
(b) ein Peptid, das ein CTL-Epitop enthält und 8 bis 13 Aminosäuren umfasst, ausgewählt aus: ist,
zur Herstellung eines Medikaments zur Verwendung in einem Verfahren zum Stimulieren einer zytotoxischen T-Lymphozyten-Reaktion auf Heptatitis B-Virus, wobei das Verfahren umfasst:
(i) das Aussetzen von zytotoxischen T-Lymphozyten eines Wirts gegenüber dem Peptid, worin die den CTL-induzierenden Peptiden ausgesetzten Wirtszellen HLA-A2-restringiert sind und worin die zytotoxischen T-Zellen aus dem Wirt entfernt wurden, bevor sie dem CTL-induzierenden Peptid ausgesetzt werden, und
(ii) das Rückführen der stimulierten CTLs an den Wirt.

11. Verfahren nach einem der Ansprüche 6 bis 9 oder Verwendung nach Anspruch 10, worin der Wirt eine akute oder chronische Hepatitis B-Infektion aufweist oder ein Hepatitis B-Träger ist.

12. Verfahren nach einem der Ansprüche 6 bis 9 oder Verwendung nach Anspruch 10, worin das CTL-induzierende Peptid prophylaktisch an die Zellen des Wirts verabreicht wird.

13. Verfahren nach einem der Ansprüche 6 bis 9 oder Anspruch 12 oder Verwendung nach Anspruch 10, worin das CTL-induzierende Peptid dem Wirt zusammen mit einem zweiten Peptid verabreicht wird, das eine T-Helfer-Reaktion auf HBV hervorruft.

14. Verfahren oder Verwendung nach Anspruch 13, worin das CTL-induzierende Peptid und das T-Helfer-induzierende Peptid verbunden sind.

## Revendications

1. Peptide induisant des CTL comprenant de huit à treize acides aminés où le peptide induisant des CTL comprend

2. Peptide induisant des CTL de la revendication 1 qui comprend de neuf à onze acides aminés.

3. Peptide de la revendication 1 ou de la revendication 2 en suspension dans un support pharmaceutiquement acceptable qui comprend un liposome.

4. Peptide induisant des CTL de l'une quelconque des revendications précédentes, où le peptide contient également un épitope T auxiliaire.

5. Peptide de l'une quelconque des revendications précédentes conjugué à un support lipidique immunogène.

6. Méthode pour la stimulation d'une réponse de lymphocytes T cytotoxiques au virus de l'hépatite B, qui consiste à exposer des lymphocytes T cytotoxiques d'un hôte à un peptide selon l'une quelconque des revendications précédentes.

7. Méthode de la revendication 6, où les cellules hôtes exposées au peptide induisant les CTL sont HLA-A2 restreintes.

8. Méthode de la revendication 7, où les cellules T cytotoxiques sont enlevées de l'hôte avant d'être exposées au peptide induisant les CTL.

9. Méthode pour stimuler une réponse de lymphocytes T cytotoxiques au virus de l'hépatite B, qui consiste à exposer des lymphocytes T cytotoxiques d'un hôte à un peptide qui contient un épitope de CTL qui comprend de huit à treize acides aminés, où le peptide induisant les CTL est sélectionné parmi un peptide selon l'une quelconque des revendications 1 à 5, ou et où les cellules hôtes exposées au peptide induisant les CTL sont HLA-A2 restreintes, et où les cellules T cytotoxiques sont retirées de l'hôte avant d'être exposées au peptide induisant les CTL.

10. Utilisation d'un peptide induisant de CTL qui est soit :
(a) un peptide selon l'une quelconque des revendications 1 à 5, ou
(b) un peptide qui contient un épitope de CTL et qui comprend de huit à treize acides aminés, sélectionnés parmi :
pour la préparation d'un médicament à utiliser dans une méthode pour la stimulation d'une réponse de lymphocytes T cytotoxiques au virus de l'hépatite B, laquelle méthode comprend :
(i) l'exposition des lymphocytes T cytotoxiques d'un hôte au peptide, où les cellules hôtes exposées aux peptides induisant des CTL sont HLA-A2 restreintes, et où les cellules T cytotoxiques sont retirées de l'hôte avant d'être exposées au peptide induisant des CTL et
(ii) le retour des CTL stimulés à l'hôte.

11. Méthode de l'une quelconque des revendications 6 à 9, ou l'utilisation de la revendication 10, où l'hôte a une infection aiguë ou chronique d'hépatite B ou est un porteur d'hépatite B.

12. Méthode selon l'une quelconque des revendications 6 à 9, ou l'utilisation de la revendication 10 où le peptide induisant des CTL est administré prophylactiquement aux cellules de l'hôte.

13. Méthode selon l'une quelconque des revendications 6 à 9, ou la revendication 12 ou l'utilisation de la revendication 10, où le peptide induisant le CTL est administré à l'hôte avec un second peptide qui élicite une réponse T auxiliaire à VHB.

14. Méthode ou utilisation de la revendication 13, où le peptide induisant les CTL et le peptide induisant T auxiliaire sont enchaînés.
